# EUROPEAN PATENT APPLICATION

(11) **EP 3 279 730 A1**
(43) Date of publication of application: **07.02.2018**
(21) Application number: 16772339.4
(22) Date of filing: 17.03.2016
(51) Int. Cl.: G03F 7/022, C08F 214/18, G03F 7/004

(54) **RADIATION-SENSITIVE COMPOSITION, AMORPHOUS FILM, AND RESIST PATTERN-FORMATION METHOD**

(30) Priority: 30.03.2015 JP 2015069988
(71) Applicant: Mitsubishi Gas Chemical Company, Inc., Tokyo 100-8324 (JP)
(72) Inventor: ECHIGO, Masatoshi, Tokyo 100-8324 (JP); TOIDA, Takumi, Hiratsuka-shi Kanagawa 254-0016 (JP); SATO, Takashi, Hiratsuka-shi Kanagawa 254-0016 (JP)
(74) Representative: Müller-Boré & Partner Patentanwälte PartG mbB
(86) International application number: PCT/JP2016/058516
(87) International publication number: WO 2016/158456

(57) **Abstract**

A radiation-sensitive composition comprising a resist base material (A), an optically active diazonaphthoquinone compound (B), and a solvent (C), wherein the content of the solvent (C) in the composition is 20 to 99% by mass, the content of components except for the solvent (C) is 1 to 80% by mass, and the resist base material (A) is a compound represented by the following formula (1): 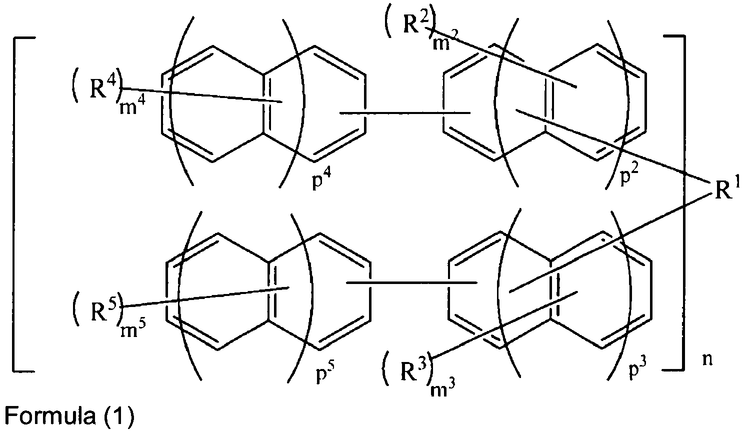 wherein R¹ is a 2n-valent group of 1 to 30 carbon atoms; R² to R⁵ are each independently an alkyl group of 1 to 10 carbon atoms, an aryl group of 6 to 10 carbon atoms, an alkenyl group of 2 to 10 carbon atoms, an alkoxy group of 1 to 30 carbon atoms, a halogen atom, a thiol group, or a hydroxyl group, wherein at least one of R⁴ and/or at least one of R⁵ is one or more kinds selected from a hydroxyl group and a thiol group; m² and m³ are each independently an integer of 0 to 8; m⁴ and m⁵ are each independently an integer of 0 to 9, wherein m⁴ and m⁵ are not 0 at the same time; n is an integer of 1 to 4; and p² to p⁵ are each independently an integer of 0 to 2.

## Description

### Technical Field

The present invention relates to a radiation-sensitive composition, an amorphous film formed using the composition, and a method for forming a resist pattern using the composition.

### Background Art

In lithography upon producing semiconductors, LCD, or solar cells, a photosensitizing agent having a quinonediazide group, such as a naphthoquinonediazide compound, and an alkali soluble resin are used. Positive type photoresists having such composition exhibit high resolving power by development with an alkaline solution and are used in the production of semiconductors such as IC and LSI or the production of circuit base materials such as LCD.

Meanwhile, for the conventional production of most advanced semiconductors, various low molecular weight resist materials have been proposed as resist base materials for offering resist patterns with higher resolution. The low molecular weight resist materials have a small molecular size because of their low molecular weights and are expected to offer resist patterns with high resolution and small roughness.

For example, an alkaline development type chemical amplification positive type radiation-sensitive composition (see e.g., Patent Literature 1 (Japanese Patent Application Laid-Open No. 2005-266741)) using a low molecular weight polynuclear polyphenolic compound as a main component, and an alkaline development type chemical amplification positive type radiation-sensitive composition (see e.g., Patent Literature 2 (Japanese Patent Application Laid-Open No. 2012-83731)) using a low molecular weight cyclic polyphenol compound as a main component have been suggested.

### Citation List

### Patent Literature

Patent Literature 1: Japanese Patent Application Laid-Open No. 2005-266741
Patent Literature 2: Japanese Patent Application Laid-Open No. 2012-83731

### Summary of Invention

### Technical Problem

As mentioned above, a large number of materials have conventionally been proposed. However, patterns derived from them have large roughness. Thus, there has been a demand for further improvement in radiation-sensitive composition.

An object of the present invention is to provide a radiation-sensitive composition which offers a good resist pattern with small roughness, an amorphous film, and a method for forming a resist pattern using the composition.

### Solution to Problem

The present inventors have, as a result of devoted examinations to solve the above problems, found out that a radiation-sensitive composition containing a resist base material having a specific chemical structure, an optically active compound, and a solvent imparts a good shape with small roughness to a resist pattern, and reached the present invention.

More specifically, the present invention is as follows.

[1] A radiation-sensitive composition comprising a resist base material (A), an optically active diazonaphthoquinone compound (B), and a solvent (C), wherein the content of the solvent (C) in the composition is 20 to 99% by mass, the content of components except for the solvent (C) is 1 to 80% by mass, and the resist base material (A) is a compound represented by the following formula (1): 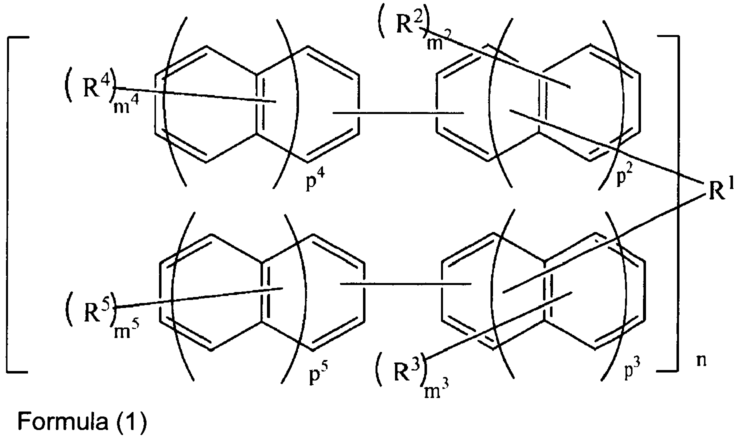 wherein R¹ is a 2n-valent group of 1 to 30 carbon atoms; R² to R⁵ are each independently an alkyl group of 1 to 10 carbon atoms, an aryl group of 6 to 10 carbon atoms, an alkenyl group of 2 to 10 carbon atoms, an alkoxy group of 1 to 30 carbon atoms, a halogen atom, a thiol group, or a hydroxyl group, wherein at least one of R⁴ and/or at least one of R⁵ is one or more kinds selected from a hydroxyl group and a thiol group; m² and m³ are each independently an integer of 0 to 8; m⁴ and m⁵ are each independently an integer of 0 to 9, wherein m⁴ and m⁵ are not 0 at the same time; n is an integer of 1 to 4; and p² to p⁵ are each independently an integer of 0 to 2.
[2] The radiation-sensitive composition according to the [1], wherein in the formula (1), at least one selected from R¹ to R⁵ is a group containing an iodine atom.
[3] The radiation-sensitive composition according to the [1] or [2], wherein in the formula (1), at least one of R² and/or at least one of R³ is one or more kinds selected from a hydroxyl group and a thiol group.
[4] The radiation-sensitive composition according to any one of the [1] to [3], wherein the compound represented by the formula (1) is a compound represented by the following formula (1a): 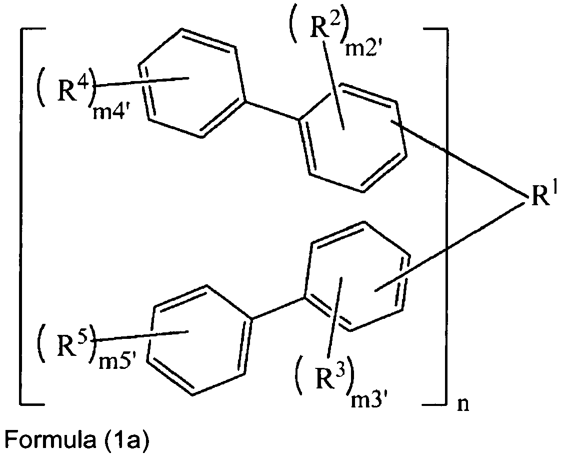 wherein R¹ to R⁵ and n are as defined in the description of the above formula (1) ; m^{2'} and m^{3'} are each independently an integer of 0 to 4; and m^{4'} and m^{5'} are each independently an integer of 0 to 5, wherein m^{4'} and m^{5'} are not 0 at the same time.
[5] The radiation-sensitive composition according to the [4], wherein the compound represented by the formula (1a) is a compound represented by the following formula (1b) : 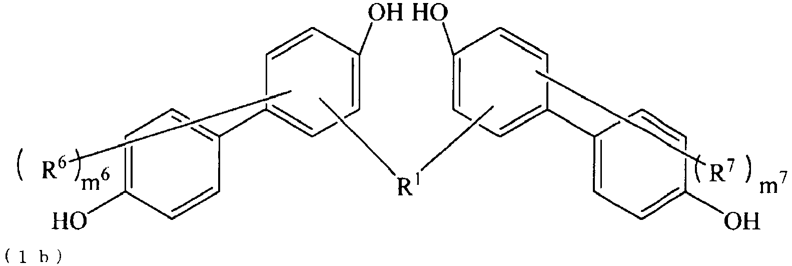 wherein R¹ is as defined in the description of the above formula (1); R⁶ and R⁷ are each independently an alkyl group of 1 to 10 carbon atoms, an aryl group of 6 to 10 carbon atoms, an alkenyl group of 2 to 10 carbon atoms, an alkoxy group of 1 to 30 carbon atoms, a halogen atom, or a thiol group, wherein at least one selected from R¹, R⁶, and R⁷ is a group containing an iodine atom; and m⁶ and m⁷ are each independently an integer of 0 to 7.
[6] The radiation-sensitive composition according to the [5], wherein the compound represented by the formula (1b) is a compound represented by the following formula (1c) : 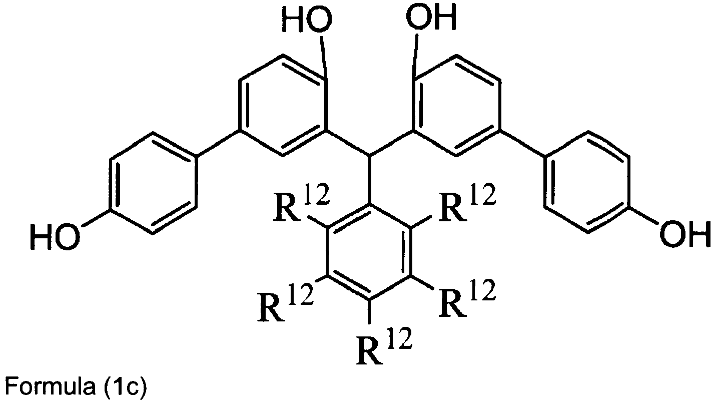 wherein R¹² are each independently a hydrogen atom, a cyano group, a nitro group, a heterocyclic group, a halogen atom, a linear aliphatic hydrocarbon group of 1 to 20 carbon atoms, a branched aliphatic hydrocarbon group of 3 to 20 carbon atoms, a cyclic aliphatic hydrocarbon group of 3 to 20 carbon atoms, an aryl group of 6 to 20 carbon atoms, an alkenyl group of 2 to 10 carbon atoms, an alkoxy group of 1 to 30 carbon atoms, a thiol group, or a hydroxyl group, wherein at least one of R¹² is a group containing an iodine atom.
[7] The radiation-sensitive composition according to the [6], wherein the compound represented by the formula (1c) is a compound represented by the following formula (1d) : 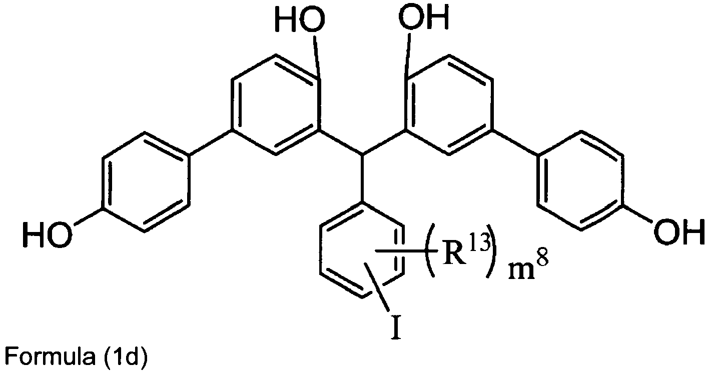 wherein R¹³ are each independently a cyano group, a nitro group, a heterocyclic group, a halogen atom, a linear aliphatic hydrocarbon group of 1 to 20 carbon atoms, a branched aliphatic hydrocarbon group of 3 to 20 carbon atoms, a cyclic aliphatic hydrocarbon group of 3 to 20 carbon atoms, an aryl group of 6 to 20 carbon atoms, an alkenyl group of 2 to 10 carbon atoms, an alkoxy group of 1 to 30 carbon atoms, a thiol group, or a hydroxyl group; and m⁸ is an integer of 0 to 4.
[8] The radiation-sensitive composition according to any one of the [1] to [7], wherein the components except for the solvent (C) comprise resist base material (A)/optically active diazonaphthoquinone compound (B)/optional component (D) at a ratio of 1 to 99/99 to 1/0 to 98 in % by mass based on the components except for the solvent (C).
[9] The radiation-sensitive composition according to any one of the [1] to [8], wherein the radiation-sensitive composition is capable of forming an amorphous film by spin coating.
[10] The radiation-sensitive composition according to the [9], wherein the dissolution rate of the amorphous film in a developing solution at 23°C is 5 angstrom/sec or less.
[11] The radiation-sensitive composition according to the [10], wherein the dissolution rate of the amorphous film irradiated with g-ray, h-ray, i-ray, KrF excimer laser, ArF excimer laser, extreme ultraviolet, electron beam, or X-ray or the amorphous film heated at 20 to 500°C, in a developing solution at 23°C is 10 angstrom/sec or more.
[12] An amorphous film obtained using the radiation-sensitive composition according to any one of the [1] to [11] .
[13] A method for forming a resist pattern, comprising the steps of: coating a substrate with the radiation-sensitive composition according to any one of the [1] to [11], thereby forming a resist film; exposing the resist film; and developing the exposed resist film.

### Advantageous Effects of Invention

The present invention can provide a radiation-sensitive composition which offers a good resist pattern with small roughness, an amorphous film, and a method for forming a resist pattern.

### Description of Embodiments

### <<Radiation-Sensitive Composition>>

The present invention provides a radiation-sensitive composition containing a resist base material (A), an optically active diazonaphthoquinone compound (B), and a solvent (C), wherein the composition contains the solvent within the range of 20 to 99% by mass and 1 to 80% by mass of components except for the solvent, and the resist base material is a compound represented by the following formula (1).

Hereinafter, the present invention will be described in detail.

### [Resist Base Material (A)]

The resist base material (A) used in the present embodiment is represented by the following formula (1):

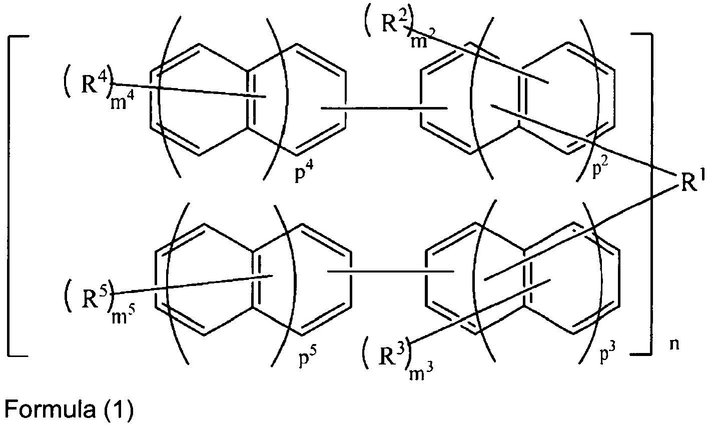

In the above formula (1), R¹ is a 2n-valent group of 1 to 30 carbon atoms, and each aromatic ring is bonded via this R¹. R¹ may contain a halogen atom such as iodine. The above 2n-valent group refers to an alkylene group of 1 to 30 carbon atoms (n = 1), an alkanetetrayl group of 1 to 30 carbon atoms (n = 2), an alkanehexayl group of 2 to 30 carbon atoms (n = 3), or an alkaneoctayl group of 3 to 30 carbon atoms (n = 4). Examples of the 2n-valent group include ones having a linear hydrocarbon group, a branched hydrocarbon group, or an alicyclic hydrocarbon group. Herein, the alicyclic hydrocarbon group also includes bridged alicyclic hydrocarbon groups. Also, the 2n-valent group may have a double bond, a heteroatom, or an aromatic group of 6 to 30 carbon atoms.

The above aromatic group may further have a cyano group, a nitro group, a heterocyclic group, a halogen atom, a linear aliphatic hydrocarbon group of 1 to 20 carbon atoms, a branched aliphatic hydrocarbon group of 3 to 20 carbon atoms, a cyclic aliphatic hydrocarbon group of 3 to 20 carbon atoms, an aryl group of 6 to 20 carbon atoms, an alkenyl group of 2 to 10 carbon atoms, an alkoxy group of 1 to 30 carbon atoms, a thiol group, or a hydroxyl group.

R² to R⁵ are each independently a group selected from the group consisting of an alkyl group of 1 to 10 carbon atoms, an aryl group of 6 to 10 carbon atoms, an alkenyl group of 2 to 10 carbon atoms, an alkoxy group of 1 to 30 carbon atoms, a halogen atom, a thiol group, and a hydroxyl group. However, at least one of R⁴ and/or at least one of R⁵ is one or more kinds selected from a hydroxyl group and a thiol group.

m² and m³ are each independently an integer of 0 to 8, and m⁴ and m⁵ are each independently an integer of 0 to 9. However, m⁴ and m⁵ are not 0 at the same time.

n is an integer of 1 to 4.

p² to p⁵ are each independently an integer of 0 to 2. For example, when p² is 0, the corresponding aromatic ring is a benzene ring. When p² is 1, the corresponding aromatic ring is a naphthalene ring. When p² is 3, the corresponding aromatic ring is a 3-membered aromatic ring such as anthracene.

In the formula (1), the alkyl group of 1 to 10 carbon atoms and the alkenyl group of 2 to 10 carbon atoms include a linear, branched, or cyclic alkyl group or alkenyl group.

The alkoxy group of 1 to 30 carbon atoms is a group composed of a group selected from a linear hydrocarbon group, a branched hydrocarbon group, or an alicyclic hydrocarbon group, an aromatic hydrocarbon group, and a group consisting of a combination of two or more thereof, and an oxygen atom. Herein, the alicyclic hydrocarbon group also includes bridged alicyclic hydrocarbon groups. Also, the alkoxy group may have a double bond, a heteroatom, or a halogen atom.

In the compound represented by the above formula (1), at least one selected from R¹ to R⁵ is preferably a group containing an iodine atom from the viewpoint of easy crosslinking, further solubility in an organic solvent, and reduction in defect of a coating film. In the present specification, the "at least one selected from R¹ to R^{5"} means "at least one group selected from R¹ to R^{5"}, and does not mean "at least one kind of group selected from R¹ to R^{5"}.

Herein, as for R¹, examples of the group containing an iodine atom include, but not particularly limited to, a linear hydrocarbon group of 1 to 30 carbon atoms substituted with an iodine atom, a branched hydrocarbon group of 3 to 30 carbon atoms substituted with an iodine atom, an alicyclic hydrocarbon group of 3 to 30 carbon atoms substituted with an iodine atom, an aromatic group of 6 to 30 carbon atoms substituted with an iodine atom, and a group having an aromatic group of 6 to 30 carbon atoms substituted with an iodine atom. A branched hydrocarbon group of 3 to 30 carbon atoms substituted with an iodine atom, an alicyclic hydrocarbon group of 3 to 30 carbon atoms substituted with an iodine atom, an aromatic group of 6 to 30 carbon atoms substituted with an iodine atom, or a group having an aromatic group of 6 to 30 carbon atoms substituted with an iodine atom is preferable, an alicyclic hydrocarbon group of 3 to 30 carbon atoms substituted with an iodine atom, an aromatic group of 6 to 30 carbon atoms substituted with an iodine atom, or a group having an aromatic group of 6 to 30 carbon atoms substituted with an iodine atom is more preferable, and a group having an aromatic group of 6 to 30 carbon atoms substituted with an iodine atom is still more preferable, from the viewpoint of heat resistance.

As for R² to R⁵, examples of the group containing an iodine atom include, but not particularly limited to, an iodine atom, a linear aliphatic alkyl group of 1 to 6 carbon atoms substituted with an iodine atom, a branched aliphatic alkyl group of 3 to 6 carbon atoms substituted with an iodine atom, a cyclic aliphatic alkyl group of 3 to 6 carbon atoms substituted with an iodine atom, and an aryl group of 6 carbon atoms substituted with an iodine atom. The group containing an iodine atom is preferably an iodine atom, a linear aliphatic alkyl group of 1 to 6 carbon atoms substituted with an iodine atom, or a branched aliphatic alkyl group of 3 to 6 carbon atoms substituted with an iodine atom, more preferably an iodine atom or a linear aliphatic alkyl group of 1 to 6 carbon atoms substituted with an iodine atom, and still more preferably an iodine atom, from the viewpoint of solubility in a safe solvent or the like.

The compound represented by the above formula (1) has high heat resistance attributed to its rigid structure, in spite of its relatively low molecular weight, and may therefore be used even under high temperature baking conditions. Furthermore, the compound represented by the above formula (1) has a relatively low molecular weight and a low viscosity and therefore facilitates uniformly and completely filling even the steps of an uneven substrate (particularly having fine space, hole pattern, etc.).

In the compound represented by the above formula (1), at least one of R² and/or at least one of R³ is preferably one or more kinds selected from a hydroxyl group and a thiol group from the viewpoint of easy crosslinking, further solubility in an organic solvent, and reduction in defect of a coating film.

The compound represented by the above formula (1) is more preferably a compound represented by the following formula (1a) from the viewpoint of the supply of raw materials:

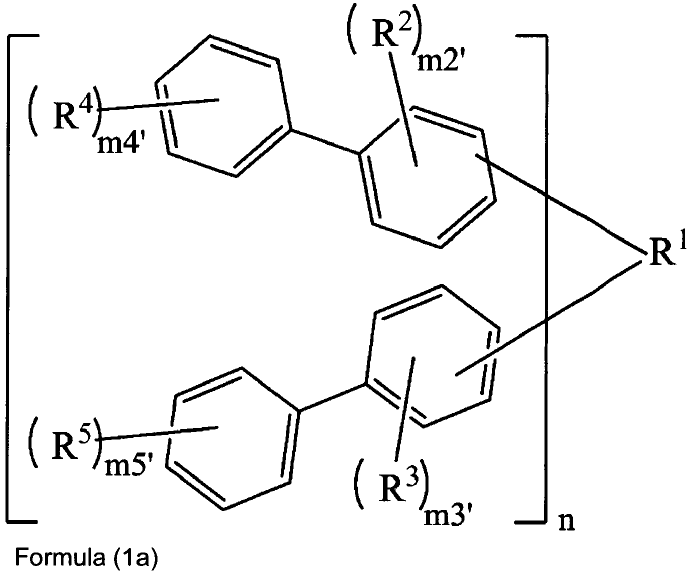

In the above formula (1a), R¹ to R⁵ and n are as defined in the description of the above formula (1). That is, at least one of R⁴ and/or at least one of R⁵ is one or more kinds selected from a hydroxyl group and a thiol group
m^{2'} and m^{3'} are each independently an integer of 0 to 4, and m^{4'} and m^{5'} are each independently an integer of 0 to 5. However, m^{4'} and m^{5'} are not 0 at the same time.

The compound represented by the above formula (1a) is still more preferably a compound represented by the following formula (1b) from the viewpoint of solubility in an organic solvent:

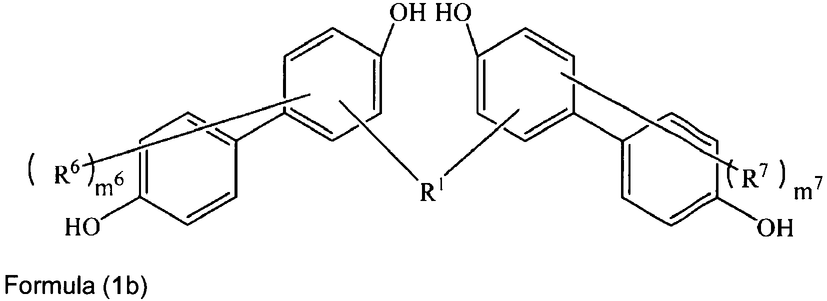

In the above formula (1b), R¹ is as defined in the description of the above formula (1). R⁶ and R⁷ are each independently an alkyl group of 1 to 10 carbon atoms, an aryl group of 6 to 10 carbon atoms, an alkenyl group of 2 to 10 carbon atoms, a halogen atom, or a thiol group. The alkyl group of 1 to 10 carbon atoms and the alkenyl group of 2 to 10 carbon atoms include a linear, branched, or cyclic alkyl group or alkenyl group. However, at least one selected from R¹, R⁶, and R⁷ is a group containing an iodine atom. In the present specification, the "at least one selected from R¹, R⁶, and R^{7"} means "at least one group selected from R¹, R⁶, and R^{7"}, and does not mean "at least one kind of group selected from R¹, R⁶, and R^{7"}. m⁶ and m⁷ are each independently an integer of 0 to 7.

The compound represented by the above formula (1b) is particularly preferably a compound represented by the following formula (1c) from the viewpoint of further solubility in an organic solvent:

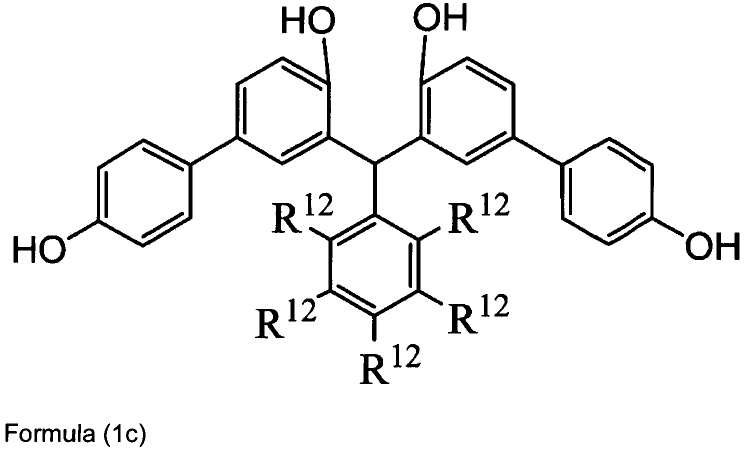

In the above formula (1c), R¹² are each independently a hydrogen atom, a cyano group, a nitro group, a heterocyclic group, a halogen atom, a linear aliphatic hydrocarbon group of 1 to 20 carbon atoms, a branched aliphatic hydrocarbon group of 3 to 20 carbon atoms, a cyclic aliphatic hydrocarbon group of 3 to 20 carbon atoms, an aryl group of 6 to 20 carbon atoms, an alkenyl group of 2 to 10 carbon atoms, an alkoxy group of 1 to 30 carbon atoms, a thiol group, or a hydroxyl group, wherein at least one of R¹² is a group containing an iodine atom, from the viewpoint of quality stabilization.

The compound represented by the above formula (1c) is particularly preferably a compound represented by the following formula (1d) from the viewpoint of easy crosslinking, further solubility in an organic solvent, and reduction in defect of a coating film:

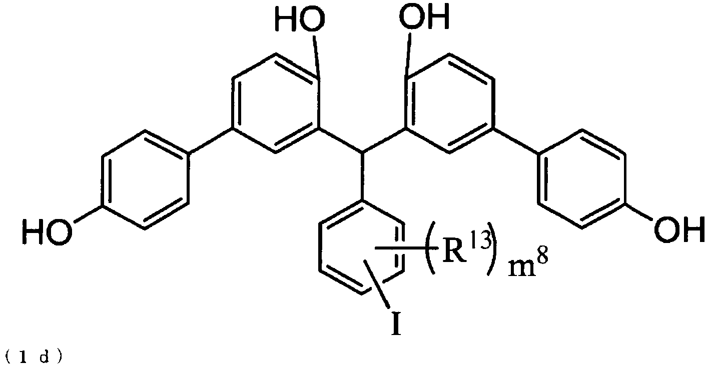

In the above formula (1d), R¹³ are each independently a cyano group, a nitro group, a heterocyclic group, a halogen atom, a linear aliphatic hydrocarbon group of 1 to 20 carbon atoms, a branched aliphatic hydrocarbon group of 3 to 20 carbon atoms, a cyclic aliphatic hydrocarbon group of 3 to 20 carbon atoms, an aryl group of 6 to 20 carbon atoms, an alkenyl group of 2 to 10 carbon atoms, an alkoxy group of 1 to 30 carbon atoms, a thiol group, or a hydroxyl group. m⁸ is an integer of 0 to 4.

Specific examples of the compound represented by the above formula (1) include, but not limited to, the followings:

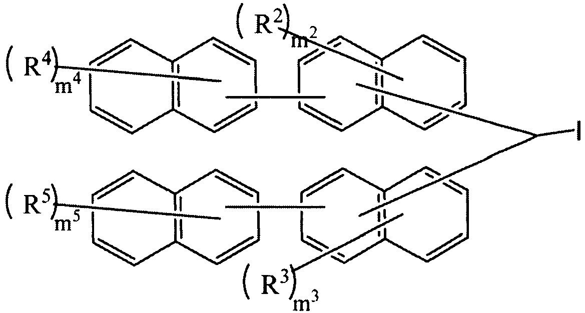

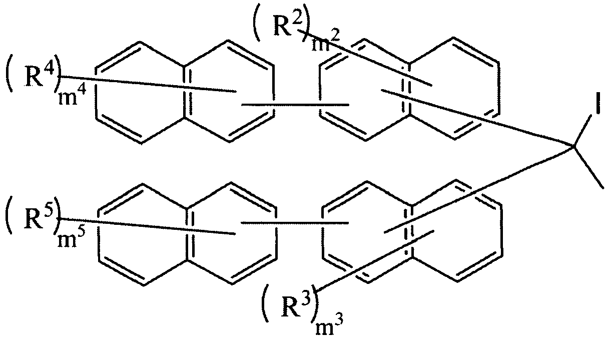

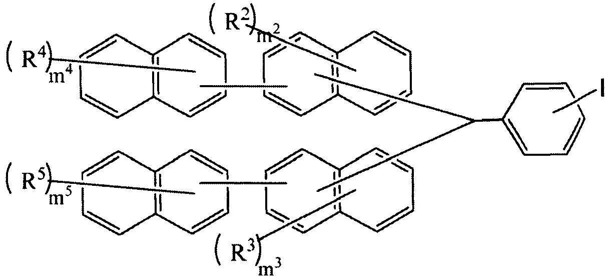

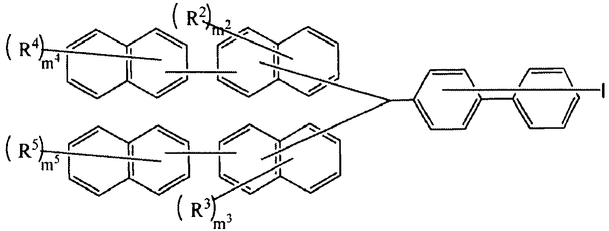

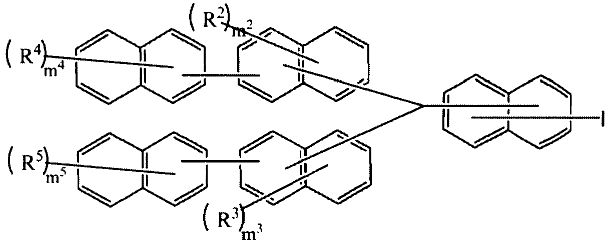

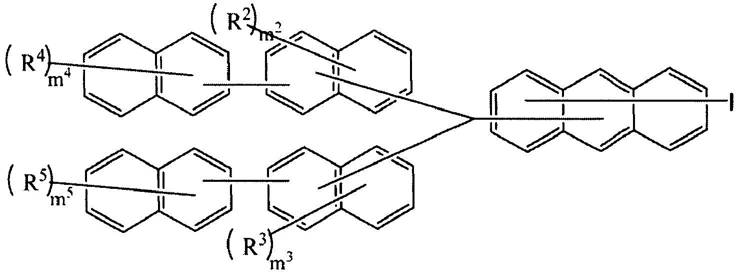

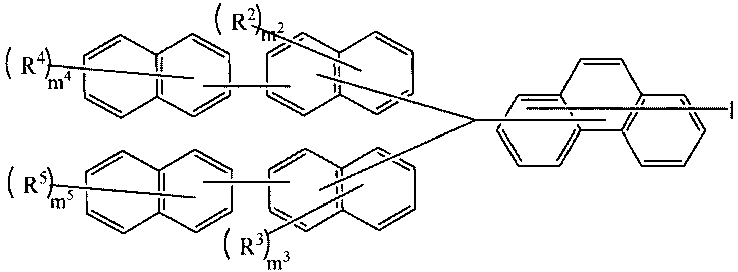

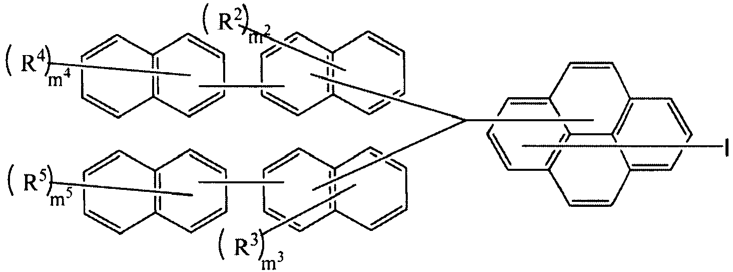

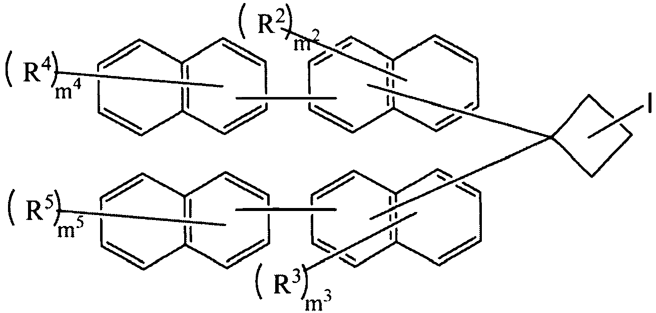

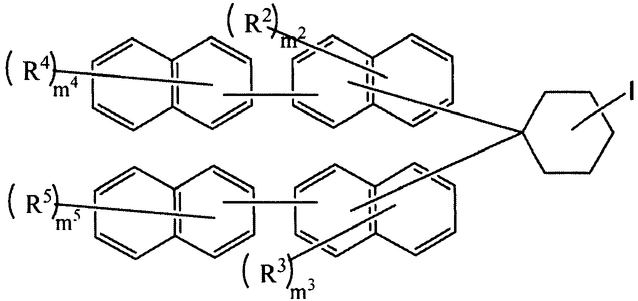

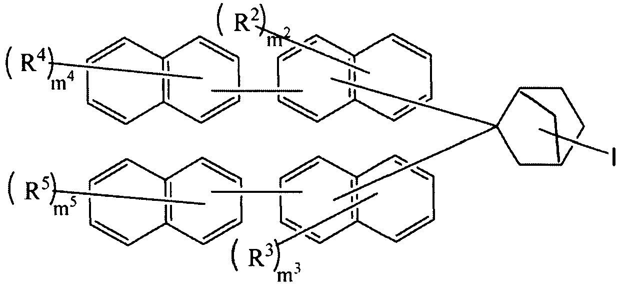

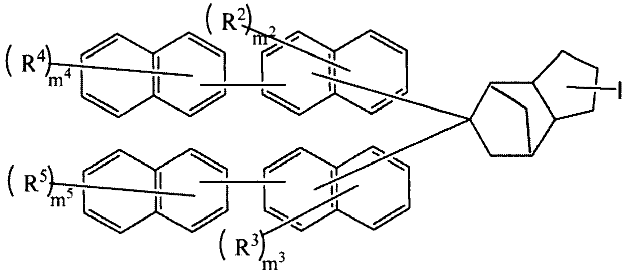

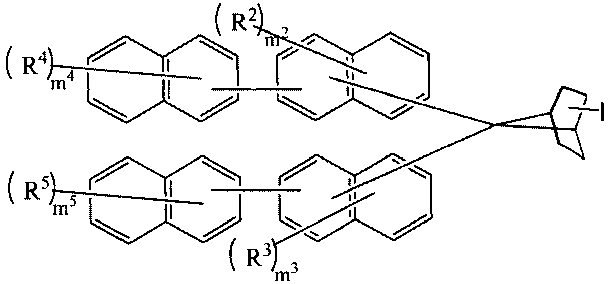

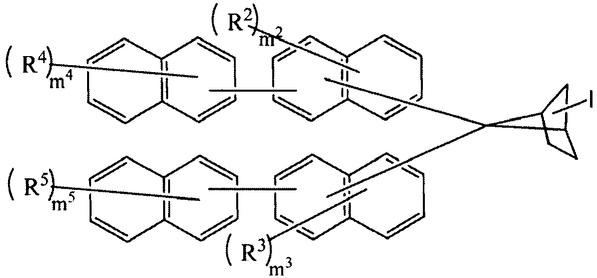

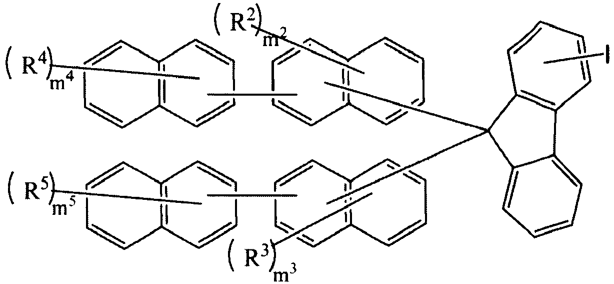

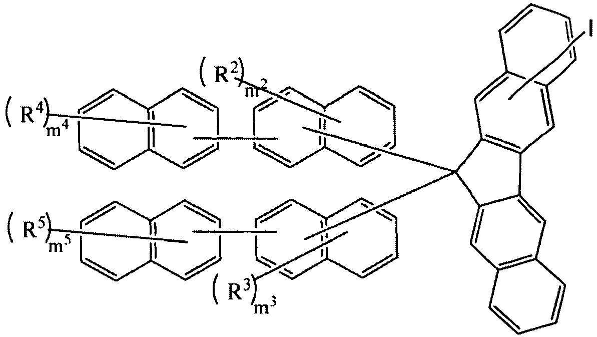

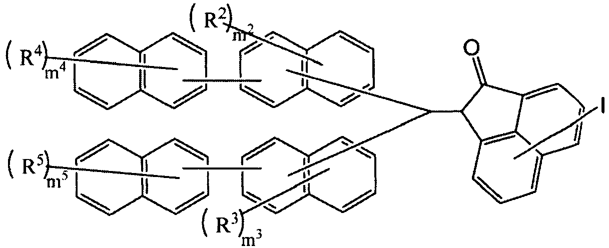

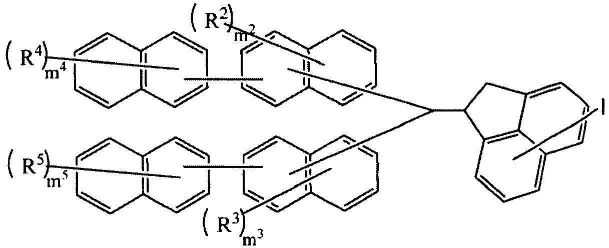

In the above compounds, R² to R⁵ and m² to m⁵ are as defined in the description of the above formula (1). However, m⁴ and m⁵ are not 0 at the same time.

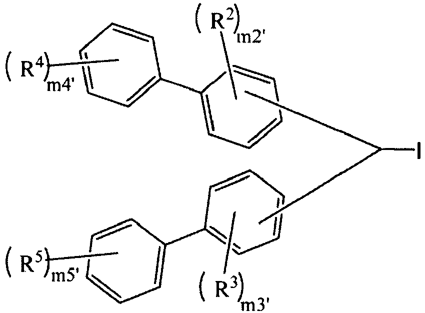

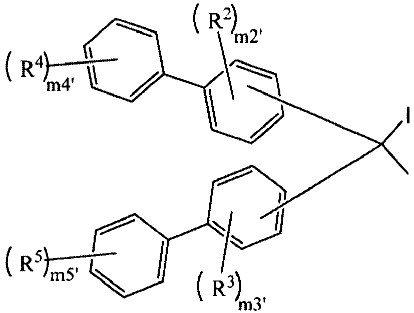

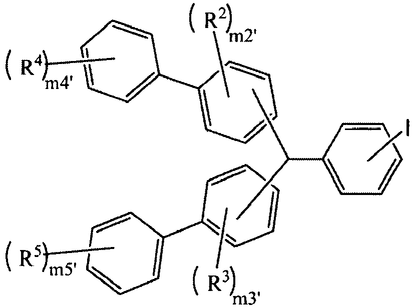

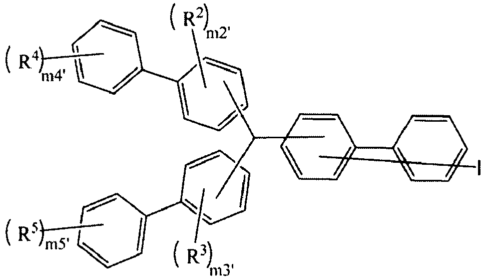

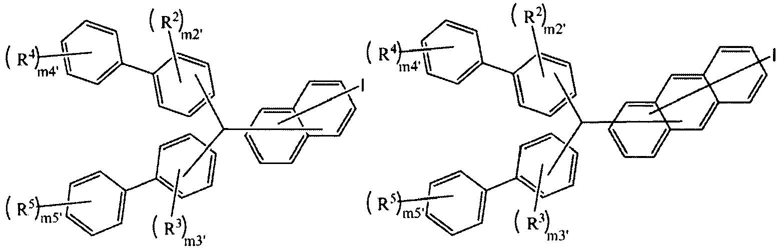

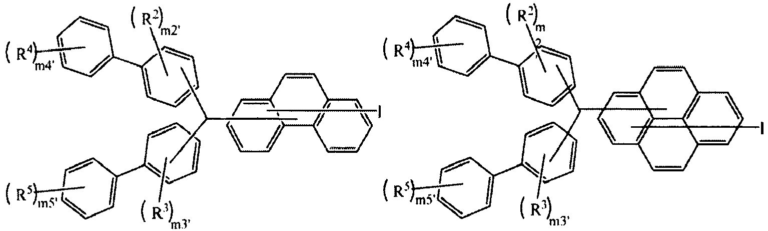

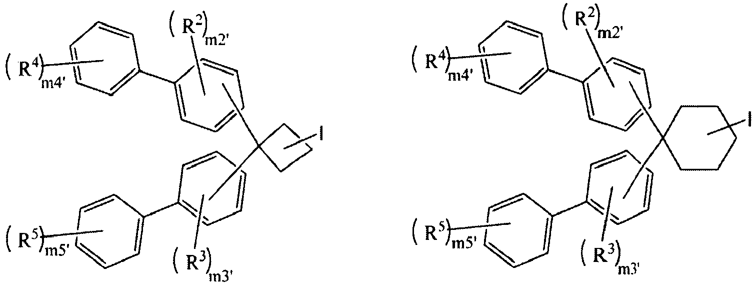

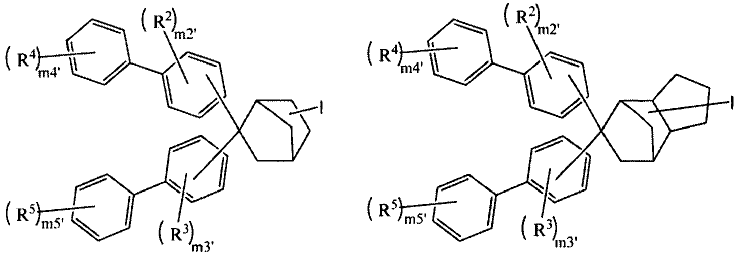

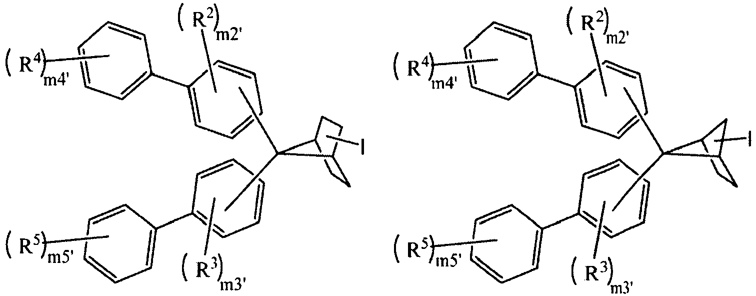

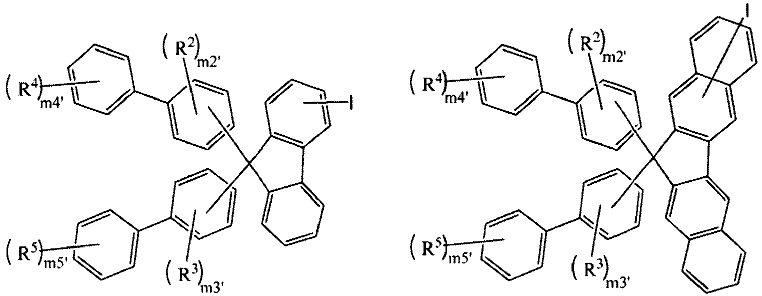

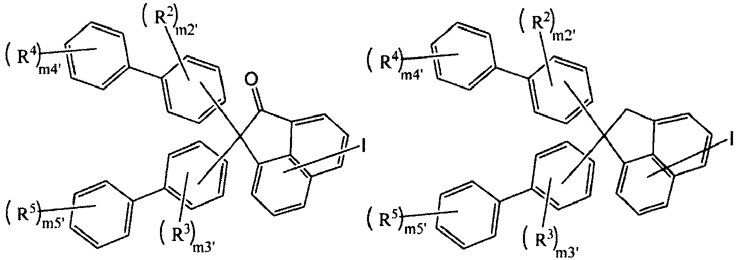

In the above compounds, R² to R⁵ are as defined in the description of the above formula (1).

m^{2'} and m^{3'} are each independently an integer of 0 to 4, and m^{4'} and m^{5'} are each independently an integer of 0 to 5. However, m^{4'} and m^{5'} are not 0 at the same time.

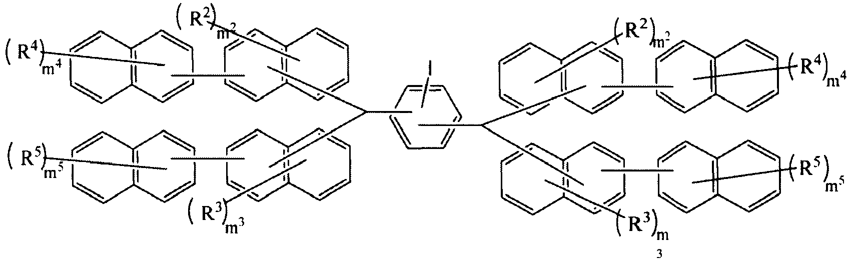

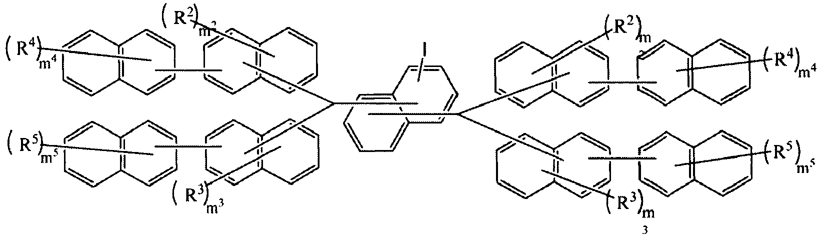

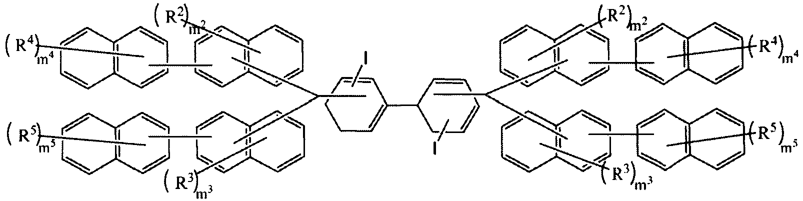

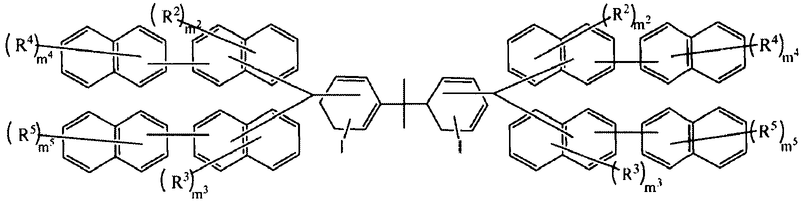

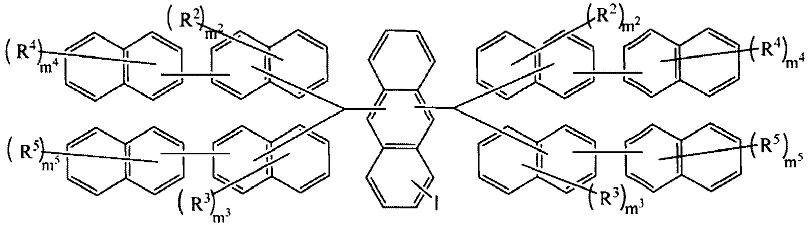

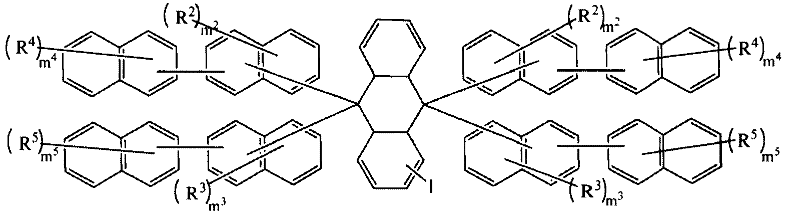

In the above compounds, R² to R⁵ and m² to m⁵ are as defined in the description of the above formula (1). However, m⁴ and m⁵ are not 0 at the same time.

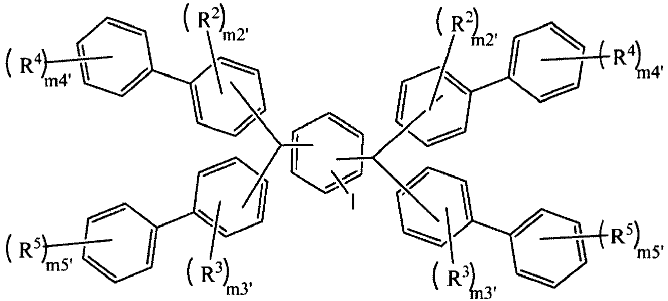

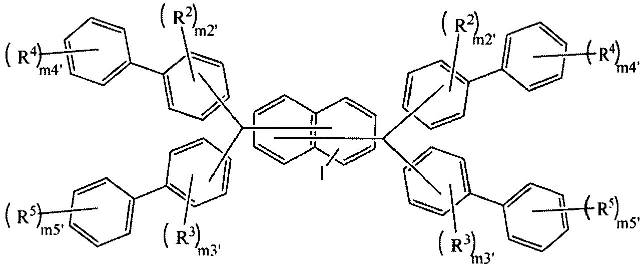

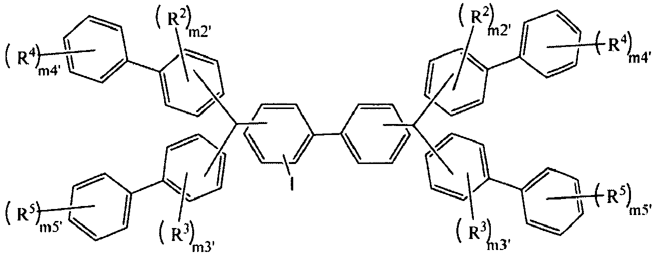

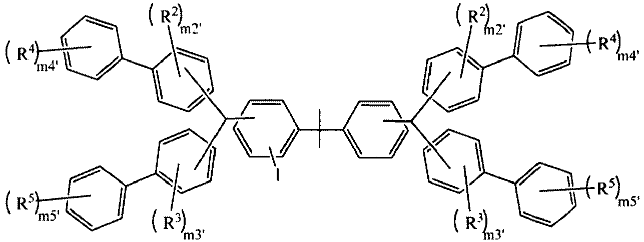

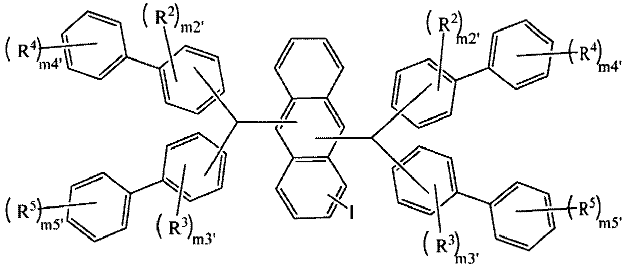

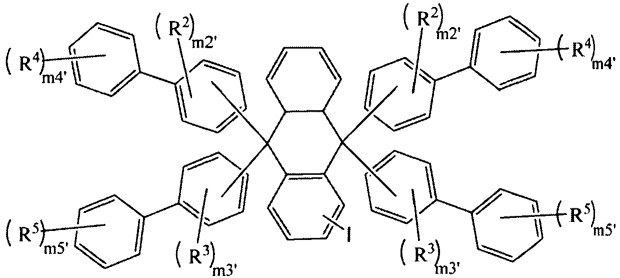

In the above compounds, R² to R⁵ are as defined in the description of the above formula (1).

m^{2'} and m^{3'} are each independently an integer of 0 to 4, and m^{4'} and m^{5'} are each independently an integer of 0 to 5. However, m^{4'} and m^{5'} are not 0 at the same time.

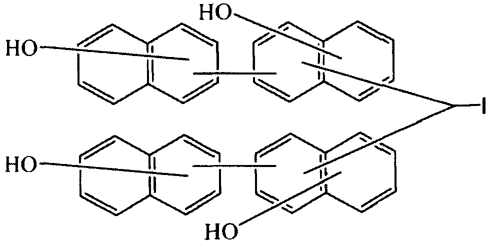

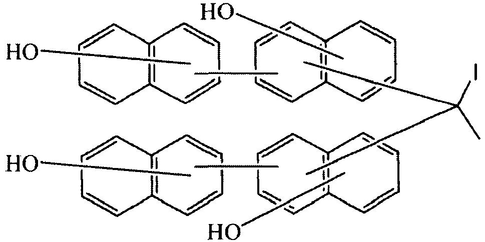

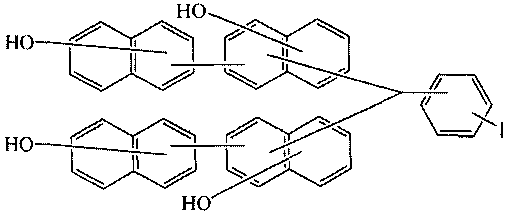

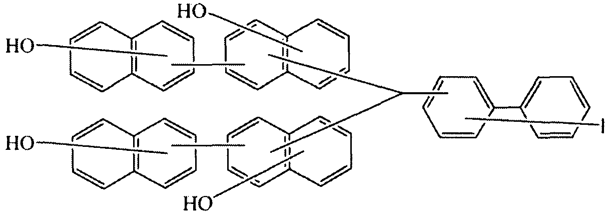

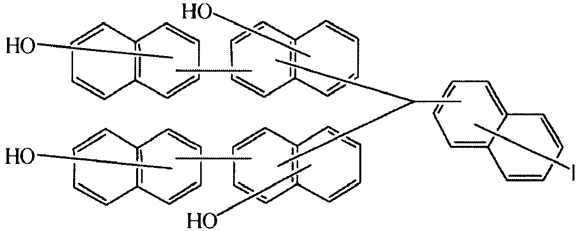

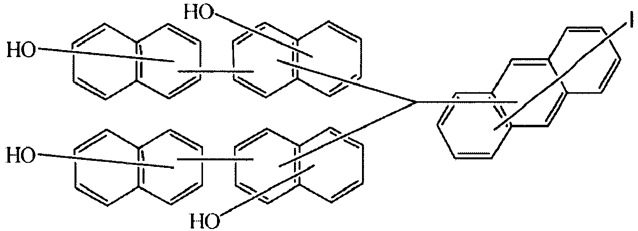

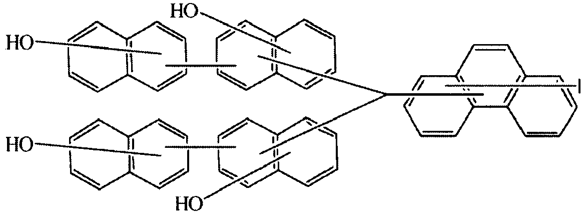

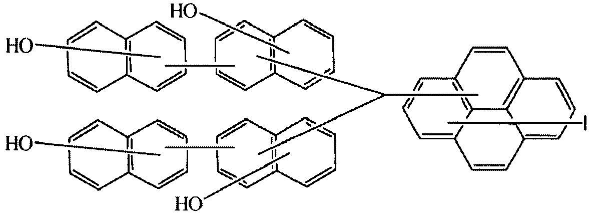

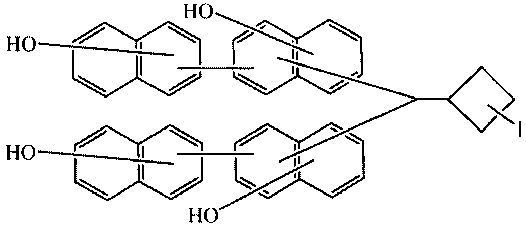

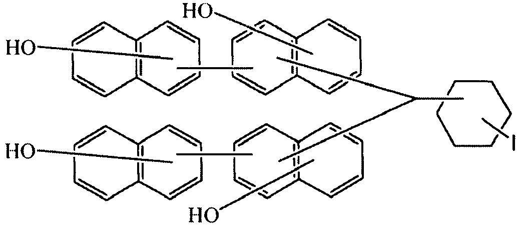

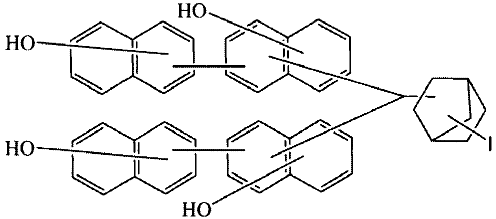

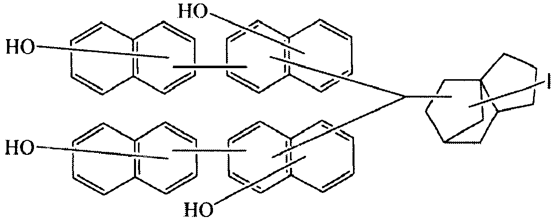

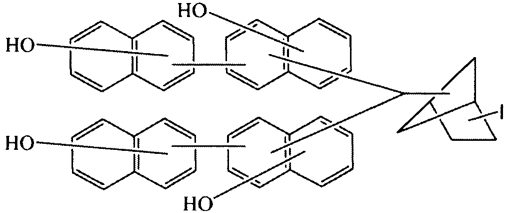

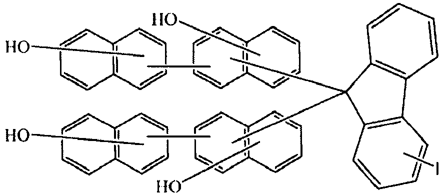

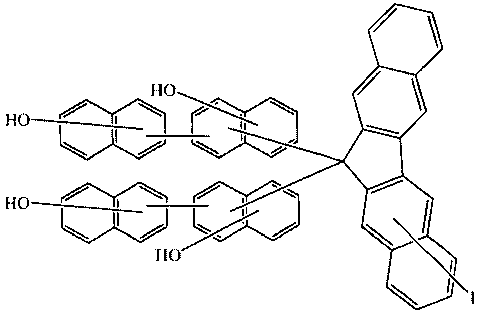

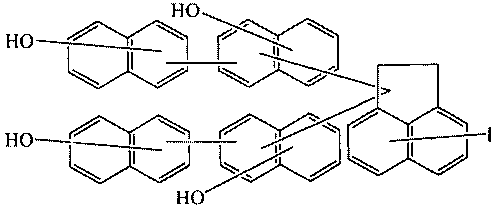

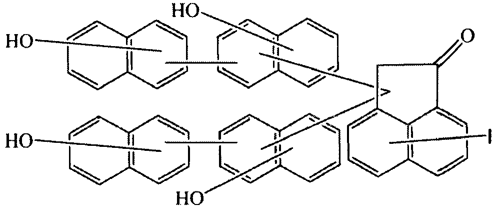

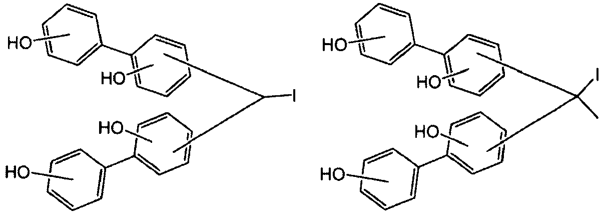

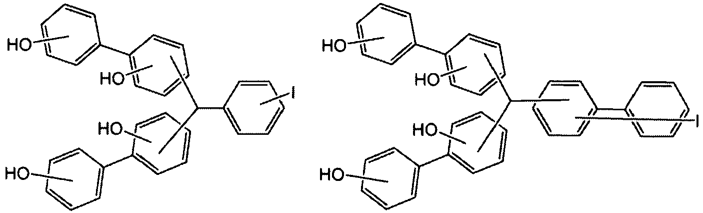

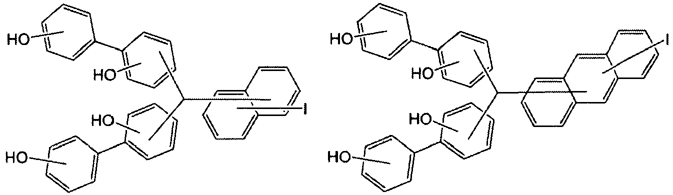

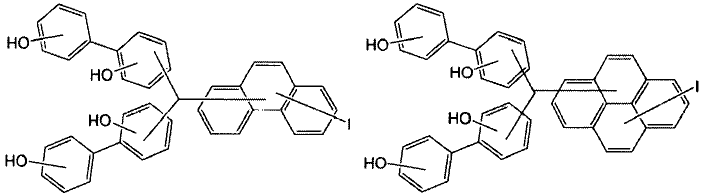

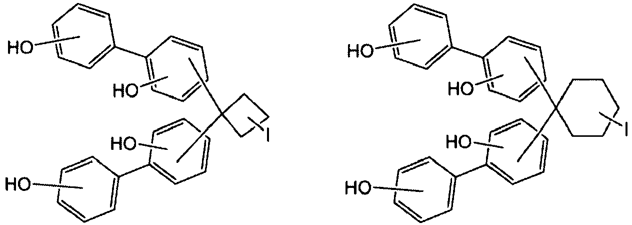

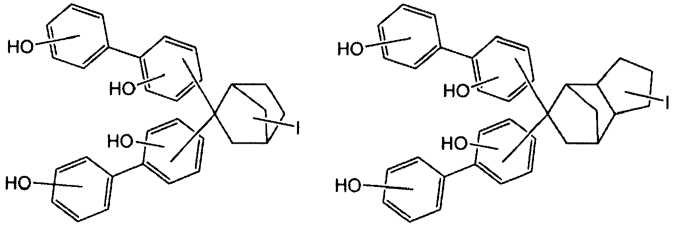

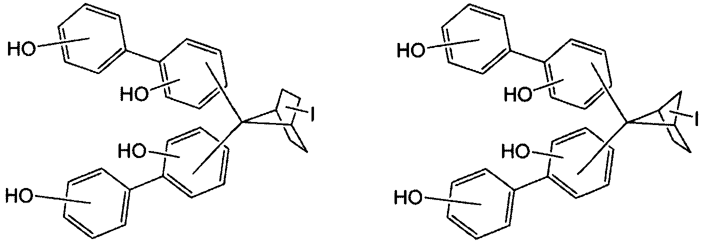

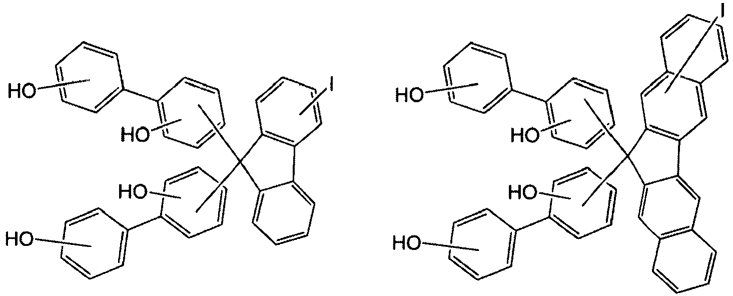

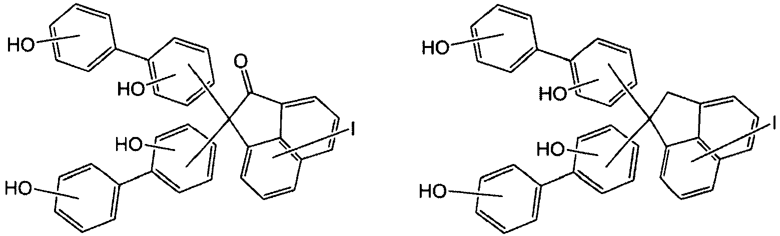

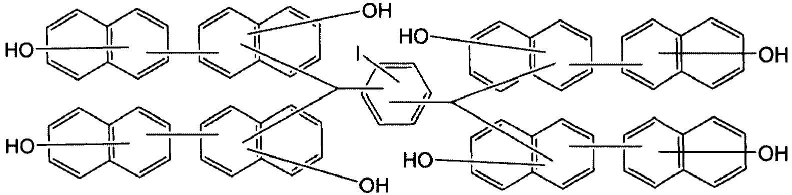

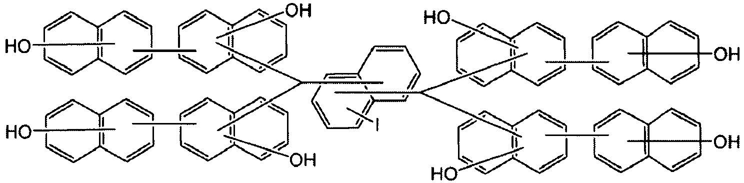

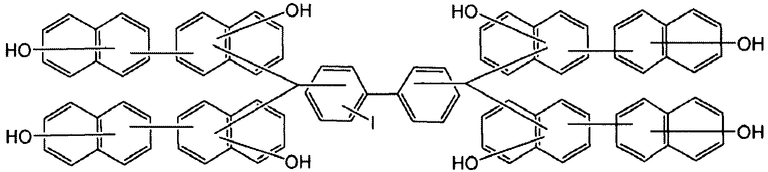

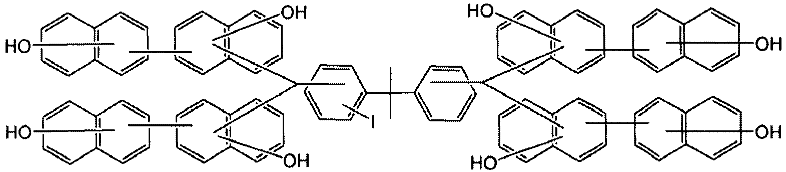

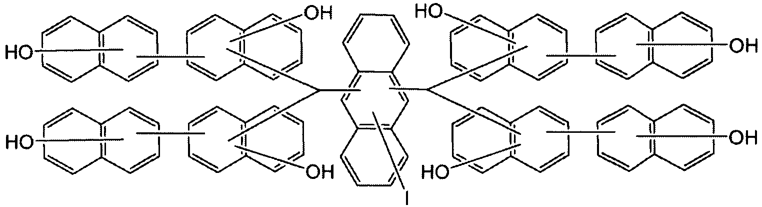

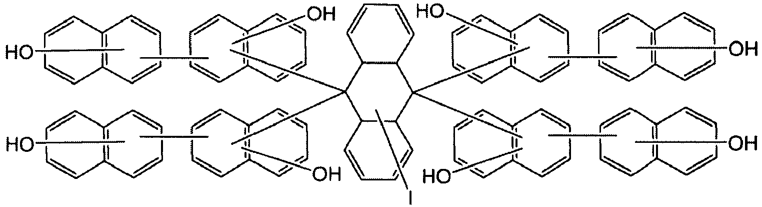

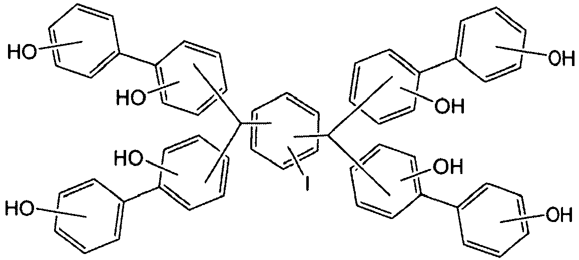

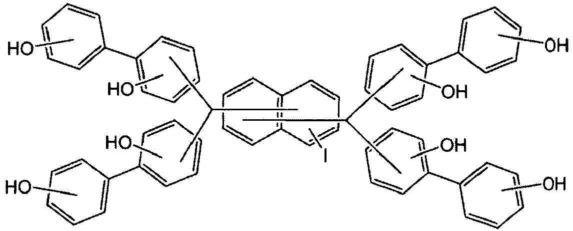

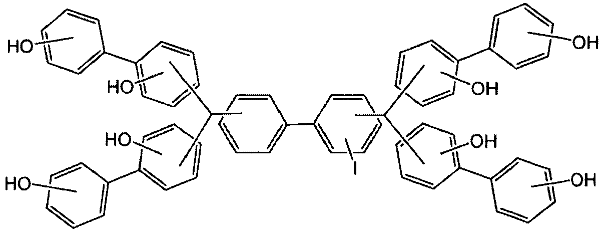

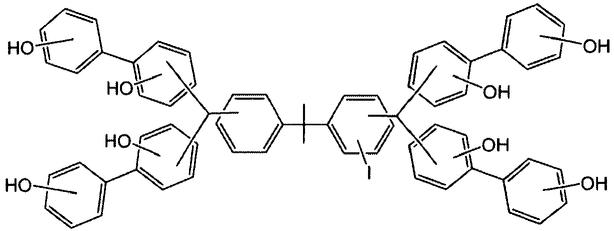

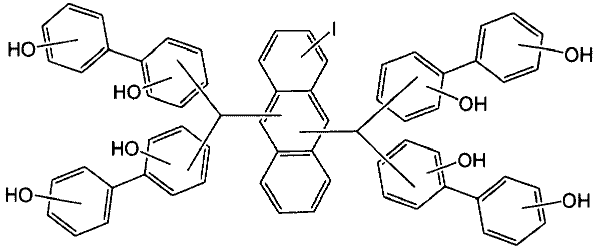

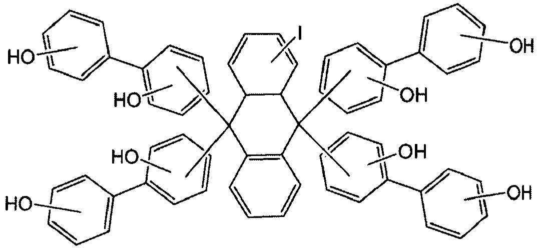

The compound represented by the formula (1) used in the present embodiment can be arbitrarily synthesized by applying a publicly known approach, and the synthesis approach is not particularly limited. The compound represented by the formula (1) can be obtained, for example, by subjecting one or more kinds of compounds (A1) selected from the group consisting of a biphenol, a bithiophenol, a binaphthol, a bithionaphthol, and a bianthraceneol, and one or more kinds of compounds (A2) selected from the group consisting of an aldehyde or a ketone and polycondensation reaction in the presence of an acid catalyst at normal pressure. If necessary, this reaction can also be carried out under increased pressure.

Examples of the biphenol as the compound (A1) include, but not particularly limited to, biphenol, methylbiphenol, and methoxybiphenol. These biphenols may be used alone as one kind or may be used in combination of two or more kinds. Among them, biphenol is more preferably used from the viewpoint of the stable supply of raw materials.

Examples of the bithiophenol as the compound (A1) include, but not particularly limited to, bithiophenol, methylbithiophenol, and methoxybithiophenol. These bithiophenols may be used alone as one kind or may be used in combination of two or more kinds. Among them, bithiophenol is more preferably used from the viewpoint of the stable supply of raw materials.

Examples of the binaphthol as the compound (A1) include, but not particularly limited to, binaphthol, methylbinaphthol, and methoxybinaphthol. These binaphthols may be used alone as one kind or may be used in combination of two or more kinds. Among them, binaphthol is more preferably used from the viewpoint of increasing a carbon atom concentration and improving heat resistance.

Examples of the bithionaphthol as the compound (A1) include, but not particularly limited to, bithionaphthol, methylbithionaphthol, and methoxybithionaphthol. These bithionaphthols may be used alone as one kind or may be used in combination of two or more kinds. Among them, bithionaphthol is more preferably used from the viewpoint of increasing a carbon atom concentration and improving heat resistance.

Examples of the bianthraceneol as the compound (A1) include, but not particularly limited to, bianthraceneol, methylbianthraceneol, and methoxybianthraceneol. These bianthraceneols may be used alone as one kind or may be used in combination of two or more kinds. Among them, bianthraceneol is more preferably used from the viewpoint of increasing a carbon atom concentration and improving heat resistance.

The aldehyde as the compound (A2) is not particularly limited. The compound is preferably, for example, a compound of 2 to 59 carbon atoms having 1 to 4 formyl groups and a group containing an iodine atom, and can be selected from an aromatic aldehyde compound and an aliphatic aldehyde compound. The aromatic aldehyde compound is preferably an aldehyde compound of 7 to 24 carbon atoms. Examples thereof include iodobenzaldehyde, methyliodobenzaldehyde, dimethyliodobenzaldehyde, ethyliodobenzaldehyde, propyliodobenzaldehyde, butyliodobenzaldehyde, ethylmethyliodobenzaldehyde, isopropylmethyliodobenzaldehyde, diethyliodobenzaldehyde, methoxyiodobenzaldehyde, iodonaphthaldehyde, iodoanthraldehyde, cyclopropyliodobenzaldehyde, cyclobutyliodobenzaldehyde, cyclopentyliodobenzaldehyde, cyclohexyliodobenzaldehyde, phenyliodobenzaldehyde, naphthyliodobenzaldehyde, adamantyliodobenzaldehyde, norbornyliodobenzaldehyde, lactyliodobenzaldehyde, isopropyliodobenzaldehyde, normal iodobenzaldehyde, bromoiodobenzaldehyde, dimethylaminoiodobenzaldehyde, hydroxyiodobenzaldehyde, dihydroxyiodobenzaldehyde, and trihydroxyiodobenzaldehyde. Iodobenzaldehyde, methyliodobenzaldehyde, dimethyliodobenzaldehyde, and ethyliodobenzaldehyde are more preferable, and iodobenzaldehyde is still more preferable. The aromatic aldehyde compound may have a linear or branched alkyl group of 1 to 4 carbon atoms, a cyano group, a hydroxyl group, halogen, or the like within the range of not deteriorating the effect of the present invention. The aromatic aldehyde compound may be used alone or in combination of two or more kinds.

The aliphatic aldehyde compound is preferably a compound of 3 to 24 carbon atoms. Examples thereof include iodopropanal, iodoisopropanal, iodobutanal, iodoisobutanal, iodo-t-butanal, iodopentanal, iodoisopentanal, iodoneopentanal, iodohexanal, iodoisohexanal, iodooctanal, iododecanal, iodododecanal, iodoundecenal, iodocyclopropanecarboxaldehyde, iodocyclobutanecarboxaldehyde, and iodocyclohexanecarboxaldehyde. Iodoisobutanal, iodo-t-butanal, iodopentanal, iodoisopentanal, iodoneopentanal, iodohexanal, iodoisohexanal, iodooctanal, iododecanal, iodododecanal, iodoundecenal, iodocyclopropanecarboxaldehyde, iodocyclobutanecarboxaldehyde, and iodocyclohexanecarboxaldehyde are more preferable, and iodooctanal, iododecanal, iodododecanal, and iodocyclohexanecarboxaldehyde are still more preferable. The aliphatic aldehyde compound may have a linear or branched alkyl group of 1 to 4 carbon atoms, a cyano group, a hydroxyl group, halogen, or the like within the range of not deteriorating the effect of the present invention. The aliphatic aldehyde compound may be used alone or in combination of two or more kinds.

The acid catalyst used in the reaction of the compound (A1) with the compound (A2) can be arbitrarily selected from publicly known ones and used without particular limitations. An inorganic acid or an organic acid is widely known as such an acid catalyst. Examples thereof include, but not particularly limited to: inorganic acids such as hydrochloric acid, sulfuric acid, phosphoric acid, hydrobromic acid, and hydrofluoric acid; organic acids such as oxalic acid, malonic acid, succinic acid, adipic acid, sebacic acid, citric acid, fumaric acid, maleic acid, formic acid, p-toluenesulfonic acid, methanesulfonic acid, trifluoroacetic acid, dichloroacetic acid, trichloroacetic acid, trifluoromethanesulfonic acid, benzenesulfonic acid, naphthalenesulfonic acid, and naphthalenedisulfonic acid; Lewis acids such as zinc chloride, aluminum chloride, iron chloride, and boron trifluoride; and solid acids such as tungstosilicic acid, tungstophosphoric acid, silicomolybdic acid, and phosphomolybdic acid. Among them, an organic acid and a solid acid are preferable from the viewpoint of production, and hydrochloric acid or sulfuric acid is preferably used from the viewpoint of production such as easy availability and handleability. The acid catalyst can be used alone as one kind or in combination of two or more kinds. The amount of the acid catalyst used, which can be arbitrarily set according to the kinds of raw materials to be used and the catalyst to be used, reaction conditions or the like, is not particularly limited and is preferably 0.01 to 100 parts by mass based on 100 parts by mass of the reaction raw materials.

In the reaction of the compound (A1) with the compound (A2), a reaction solvent may be used. The reaction solvent is not particularly limited as long as the reaction of the aldehyde or the ketone to be used with the biphenol, the bithiophenol, the binaphthol, the bithionaphthol, or the bianthraceneol proceeds. The reaction solvent can be arbitrarily selected from publicly known ones and used. Examples of the above reaction solvent include ethyl acetate, propyl acetate, butyl acetate, 4-butyrolactone, ethylene glycol, propylene glycol, methanol, ethanol, propanol, butanol, tetrahydrofuran, dioxane, ethylene glycol dimethyl ether, ethylene glycol diethyl ether, propylene glycol monomethyl ether, propylene glycol monoethyl ether, propylene glycol monomethyl ether acetate, and mixed solvents thereof. The solvent can be used alone as one kind or in combination of two or more kinds.

The amount of the reaction solvent used, which can be arbitrarily set according to the kinds of raw materials to be used and the catalyst to be used, reaction conditions or the like, is not particularly limited and is preferably within the range of 0 to 2000 parts by mass based on 100 parts by mass of the reaction raw materials. The reaction temperature in the reaction of the compound (A1) with the compound (A2), which can be arbitrarily selected according to the reactivity of the reaction raw materials, is not particularly limited and is usually within the range of 10 to 200°C. For obtaining the compound represented by the formula (1) according to the present embodiment, a higher reaction temperature is more preferable. Specifically, the range of 60 to 200°C is preferable.

The reaction method can be arbitrarily selected from publicly known approaches and used without particular limitations. The method includes a method of charging the biphenol, the bithiophenol, the binaphthol, the bithionaphthol, or the bianthraceneol, the aldehyde or the ketone, and the catalyst in one portion, and a method of dropping the biphenol, the bithiophenol, the binaphthol, the bithionaphthol, or the bianthraceneol, and the aldehyde in the presence of the catalyst. After the polycondensation reaction terminates, the obtained compound can be isolated according to a routine method without particular limitations. For example, the objective compound can be obtained by adopting a general approach of, for example, elevating the temperature of the reaction vessel to 130 to 230°C in order to remove unreacted raw materials, catalyst, etc. present in the system, and removing volatile portions at about 1 to 50 mmHg.

The reaction of the compound (A1) with the compound (A2) proceeds under preferable conditions involving using 1.0 mol to an excess of the biphenol, the bithiophenol, the binaphthol, the bithionaphthol, or the bianthraceneol and 0.001 to 1 mol of the acid catalyst based on 1 mol of the aldehyde or the ketone, and reacting them at 50 to 150°C at normal pressure for about 20 minutes to 100 hours.

The target component can be isolated by a publicly known method after the reaction terminates. An exemplary method can involve concentrating the reaction solution, precipitating the reaction product by the addition of pure water, cooling the reaction solution to room temperature, then separating the precipitates by filtration, filtering and drying the obtained solid matter, then separating and purifying the solid matter from by-products by column chromatography, and distilling off the solvent, followed by filtration and drying to obtain the compound represented by the above formula (1) which is the objective compound.

Two or more kinds of at least one of the above compound (A1) or the above compound (A2) are more preferably used for the resist base material (A) from the viewpoint of improving the solubility of the resulting resist base material (A) in a semiconductor safe solvent.

If required, the compound thus obtained may be purified in order to improve the purity of the resist base material (A) and to reduce the amount of residual metals. If an acid catalyst and a promoter remain, the storage stability of a radiation-sensitive composition generally tends to be reduced. Alternatively, if a basic catalyst remains, the sensitivity of a radiation-sensitive composition generally tends to be reduced. Therefore, the above purification may be conducted for the purpose of reducing them.

The above purification can be conducted by a publicly known method as long as the resist base material (A) is not denatured. Examples thereof include, but not particularly limited to, a method involving washing with water, a method involving washing with an acidic aqueous solution, a method involving washing with a basic aqueous solution, a method involving treatment with an ion exchange resin, and a method involving treatment by silica gel column chromatography. These purification methods are more preferably conducted in combination of two or more kinds.

The above acidic aqueous solution, basic aqueous solution, ion exchange resin, and silica gel column chromatography may be arbitrarily selected from optimal ones according to the metals to be removed, the amounts or types of acidic compounds and basic compounds, the type of the resist base material to be purified or the like. Examples of the acidic aqueous solution include aqueous solutions of hydrochloric acid, nitric acid, or acetic acid having a concentration of 0.01 to 10 mol/L. Examples of the basic aqueous solution include aqueous solutions of ammonia having a concentration of 0.01 to 10 mol/L. Examples of the ion exchange resin include cationic exchange resins, for example, "Amberlyst 15J-HG Dry" manufactured by Organo Corp.

The compound thus purified may be dried. The drying can be conducted by a publicly known method. Examples thereof include, but not particularly limited to, a method of conducting drying in vacuum or drying in hot air under conditions of not denaturing the cyclic compound.

The radiation-sensitive composition of the present embodiment containing the resist base material (A) can form an amorphous film by spin coating. Also, the radiation-sensitive composition can be applied to a general semiconductor production process.

The resist base material (A) according to the present embodiment is used in combination with the optically active diazonaphthoquinone compound (B) mentioned later and is useful as a base material for positive type resists that becomes a compound easily soluble in a developing solution by irradiation with g-ray, h-ray, i-ray, KrF excimer laser, ArF excimer laser, extreme ultraviolet, electron beam, or X-ray. Although the properties of the resist base material are not largely altered by g-ray, h-ray, i-ray, KrF excimer laser, ArF excimer laser, extreme ultraviolet, electron beam, or X-ray, the optically active diazonaphthoquinone compound (B) poorly soluble in a developing solution is converted to an easily soluble compound so that a resist pattern can be formed in a development step. Since the resist base material according to the present embodiment is a low molecular weight compound, the obtained resist pattern has very small roughness. Use of the resist base material having a group containing an iodine atom which is a preferable form increases the ability to absorb radiation such as electron beam, extreme ultraviolet (EUV), or X-ray. As a result, this enables the enhancement of the sensitivity of the radiation-sensitive composition using the resist base material of the present invention, which is very preferable.

The glass transition temperature of the resist base material (A) according to the present embodiment is preferably 100°C or more, more preferably 120°C or more, still more preferably 140°C or more, and particularly preferably 150°C or more. When the glass transition temperature falls within the above range, the resulting radiation-sensitive composition has heat resistance capable of maintaining a pattern shape in a semiconductor lithography process, and improves performance such as high resolution.

The heat of crystallization determined by the differential scanning calorimetry of the glass transition temperature of the resist base material (A) according to the present embodiment is preferably less than 20 J/g. (Crystallization temperature) - (Glass transition temperature) is preferably 70°C or more, more preferably 80°C or more, still more preferably 100°C or more, and particularly preferably 130°C or more. When the heat of crystallization is less than 20 J/g or (Crystallization temperature) - (Glass transition temperature) falls within the above range, the radiation-sensitive composition easily forms an amorphous film by spin coating, can maintain film formability necessary for a resist over a long period, and can improve resolution.

In the present embodiment, the above heat of crystallization, crystallization temperature, and glass transition temperature can be determined by differential scanning calorimetry using "DSC/TA-50WS" manufactured by Shimadzu Corp. For the above heat of crystallization, for example, about 10 mg of a sample is placed in an unsealed container made of aluminum, and the temperature is raised to the melting point or more at a temperature increase rate of 20°C/min in a nitrogen gas stream (50 mL/min). After quenching, again the temperature is raised to the melting point or more at a temperature increase rate of 20°C/min in a nitrogen gas stream (30 mL/min). After further quenching, again the temperature is raised to 400°C at a temperature increase rate of 20°C/min in a nitrogen gas stream (30 mL/min). The temperature at the middle point (where the specific heat is changed into the half) of steps in the baseline shifted in a step-like pattern is defined as the glass transition temperature (Tg). The temperature of the subsequently appearing exothermic peak is defined as the crystallization temperature. The heat can be determined from the area of a region surrounded by the exothermic peak and the baseline and defined as the heat of crystallization.

The resist base material (A) according to the present embodiment is preferably low sublimable at 100°C or lower, preferably 120°C or lower, more preferably 130°C or less, still more preferably 140°C or less, and particularly preferably 150°C or less at normal pressure. The "low sublimability" means that in thermogravimetry, weight reduction when the resist base material is kept at a predetermined temperature for 10 minutes is 10%, preferably 5%, more preferably 3%, still more preferably 1%, and particularly preferably 0.1% or less. The low sublimability of the resist base material (A) can prevent an exposure apparatus from being contaminated by outgassing upon exposure. In addition, a good pattern shape with low roughness can be obtained.

The resist base material according to the present embodiment dissolves at preferably 1% by mass or more, more preferably 5% by mass or more, and still more preferably 10% by mass or more at 23°C in a solvent that is selected from propylene glycol monomethyl ether acetate (PGMEA), propylene glycol monomethyl ether (PGME), cyclohexanone (CHN), cyclopentanone (CPN), 2-heptanone, anisole, butyl acetate, ethyl propionate, and ethyl lactate and exhibits the highest ability to dissolve the compound. Particularly preferably, the resist base material dissolves at 20% by mass or more at 23°C in a solvent that is selected from PGMEA, PGME, and CHN and exhibits the highest ability to dissolve the compound. Particularly preferably, the resist base material dissolves at 20% by mass or more at 23°C in PGMEA. When the above conditions are met, the resist base material according to the present embodiment is easily used in a semiconductor production process at a full production scale.

### [Optically Active Diazonaphthoquinone Compound (B)]

The optically active diazonaphthoquinone compound (B) according to the present embodiment is a diazonaphthoquinone substance including a polymer or non-polymer optically active diazonaphthoquinone compound and is not particularly limited as long as it is generally used as a photosensitive component in positive type resist compositions. One kind or two or more kinds can be optionally selected and used.

As the optically active diazonaphthoquinone compound (B) according to the present embodiment, a compound obtained by reacting naphthoquinonediazide sulfonic acid chloride, benzoquinonediazide sulfonic acid chloride, or the like with a low molecular weight compound or a high molecular weight compound having a functional group condensable with these acid chlorides can be preferably used.

Preferable examples of the above acid chloride such as naphthoquinonediazide sulfonic acid chloride or benzoquinonediazide sulfonic acid chloride include 1,2-naphthoquinonediazide-5-sulfonyl chloride and 1,2-naphthoquinonediazide-4-sulfonyl chloride.

Examples of the "above functional group condensable with the acid chlorides" include a hydroxyl group and an amino group. Particularly, a hydroxyl group is preferable. Examples of the compound containing a hydroxyl group condensable with the acid chlorides can include hydroquinone, resorcin, hydroxybenzophenones such as 2,4-dihydroxybenzophenone, 2,3,4-trihydroxybenzophenone, 2,4,6-trihydroxybenzophenone, 2,4,4'-trihydroxybenzophenone, 2,3,4,4'-tetrahydroxybenzophenone, 2,2',4,4'-tetrahydroxybenzophenone, and 2,2',3,4,6'-pentahydroxybenzophenone, hydroxyphenylalkanes such as bis(2,4-dihydroxyphenyl)methane, bis(2,3,4-trihydroxyphenyl)methane, and bis(2,4-dihydroxyphenyl)propane, and hydroxytriphenylmethanes such as 4,4',3",4"-tetrahydroxy-3,5,3',5'-tetramethyltriphenylmethane and 4,4',2",3",4"-pentahydroxy-3,5,3',5'-tetramethyltriphenylmethane.

### [Solvent (C)]

The radiation-sensitive composition of the present embodiment contains a solvent (C) together with the above resist base material (A) and the above optically active diazonaphthoquinone compound (B). The solvent is usually prepared by dissolving each component in the solvent upon use into a homogenous solution, and then if required, filtering through a filter or the like with a pore diameter of about 0.2 µm, for example.

Examples of the solvent used in the present embodiment can include, but not particularly limited to, ethylene glycol monoalkyl ether acetates such as ethylene glycol monomethyl ether acetate, ethylene glycol monoethyl ether acetate, ethylene glycol mono-n-propyl ether acetate, and ethylene glycol mono-n-butyl ether acetate; ethylene glycol monoalkyl ethers such as ethylene glycol monomethyl ether and ethylene glycol monoethyl ether; propylene glycol monoalkyl ether acetates such as propylene glycol monomethyl ether acetate (PGMEA), propylene glycol monoethyl ether acetate, propylene glycol mono-n-propyl ether acetate, and propylene glycol mono-n-butyl ether acetate; propylene glycol monoalkyl ethers such as propylene glycol monomethyl ether (PGME) and propylene glycol monoethyl ether; ester lactates such as methyl lactate, ethyl lactate, n-propyl lactate, n-butyl lactate, and n-amyl lactate; aliphatic carboxylic acid esters such as methyl acetate, ethyl acetate, n-propyl acetate, n-butyl acetate, n-amyl acetate, n-hexyl acetate, methyl propionate, and ethyl propionate; other esters such as methyl 3-methoxypropionate, ethyl 3-methoxypropionate, methyl 3-ethoxypropionate, ethyl 3-ethoxypropionate, methyl 3-methoxy-2-methylpropionate, 3-methoxybutylacetate, 3-methyl-3-methoxybutylacetate, butyl 3-methoxy-3-methylpropionate, butyl 3-methoxy-3-methylbutyrate, methyl acetoacetate, methyl pyruvate, and ethyl pyruvate; aromatic hydrocarbons such as toluene and xylene; ketones such as 2-heptanone, 3-heptanone, 4-heptanone, cyclopentanone, and cyclohexanone (CHN); amides such as N,N-dimethylformamide, N-methylacetamide, N,N-dimethylacetamide, and N-methylpyrrolidone; and lactones such as γ-lactone. These solvents can be used alone or in combination of two or more kinds.

### [Radiation-Sensitive Composition]

The radiation-sensitive composition of the present embodiment contains the above resist base material (A), the above optically active diazonaphthoquinone compound (B), and the above solvent (C). The content of the solvent (C) in the composition is 20 to 99% by mass, and the content of components except for the solvent (C) is 1 to 80% by mass. The content of the components except for the solvent (C) is preferably 1 to 50% by mass, more preferably 2 to 40% by mass, and still more preferably 2 to 10% by mass, from the viewpoint of the resolution of a resist pattern. The total of the solvent (C) and the components except for the solvent (C) is 100% by mass.

The content of the resist base material (A) is preferably 1 to 99% by mass of the total mass of the components except for the solvent (C) (summation of the resist base material (A), the optically active diazonaphthoquinone compound (B), and an optionally used component (D) such as other components, hereinafter the same), more preferably 5 to 95% by mass, still more preferably 10 to 90% by mass, and particularly preferably 25 to 75% by mass. The above content ratio of the resist base material (A) can produce a pattern with high sensitivity and small roughness.

The content of the optically active diazonaphthoquinone compound (B) is preferably 1 to 99% by mass of the total mass of the components except for the solvent, more preferably 5 to 95% by mass, still more preferably 10 to 90% by mass, and particularly preferably 25 to 75% by mass. The above content ratio of the optically active diazonaphthoquinone compound (B) can produce a pattern with high sensitivity and small roughness.

The radiation-sensitive composition of the present embodiment can form an amorphous film by spin coating. The dissolution rate of the amorphous film formed by spin coating with the radiation-sensitive composition of the present embodiment in a developing solution at 23°C is preferably 5 angstrom/sec or less, more preferably 0.05 to 5 angstrom/sec, and still more preferably 0.0005 to 5 angstrom/sec. When the dissolution rate is 5 angstrom/sec or less, the amorphous film is insoluble in a developing solution, and can produce a resist. When the amorphous film has the dissolution rate of 0.0005 angstrom/sec or more, the resolution may be improved. It is presumed that this is because due to the change in the solubility before and after exposure of the compound, contrast at the interface between the exposed portion being dissolved in a developing solution and the unexposed portion not being dissolved in a developing solution is increased. Moreover, reduction effects of roughness and defect are obtained.

The dissolution rate of the portion exposed by radiation such as g-ray, h-ray, i-ray, KrF excimer laser, ArF excimer laser, extreme ultraviolet, electron beam or X-ray, of the amorphous film formed by spin coating with the radiation-sensitive composition of the present embodiment, in a developing solution at 23°C, or the dissolution rate of the portion of the above amorphous film after heating at 20 to 500°C, in a developing solution at 23°C is preferably 10 angstrom/sec or more, more preferably 10 to 10000 angstrom/sec, and still more preferably 100 to 1000 angstrom/sec. When the dissolution rate is 10 angstrom/sec or more, the above portion dissolves in a developing solution, and can produce a resist. When the above portion has the dissolution rate of 10000 angstrom/sec or less, the resolution may be improved. It is presumed that this is because the micro surface portion of the compound dissolves and roughness is reduced. Moreover, reduction effects of defect are obtained.

### [Other Optional Component (D)]

To the radiation-sensitive composition of the present embodiment, within the range of not inhibiting the purpose of the present invention, if required, as the other component (D), one kind or two kinds or more of various additive agents such as an acid generating agent, an acid crosslinking agent, an acid diffusion controlling agent, a dissolution promoting agent, a dissolution controlling agent, a sensitizing agent, a surfactant and an organic carboxylic acid or an oxo acid of phosphor, or derivative thereof can be added.

### (1) Acid Generating Agent

The radiation-sensitive composition of the present embodiment may contain one or more acid generating agent generating an acid directly or indirectly by irradiation of any radiation selected from visible light, ultraviolet, excimer laser, electron beam, extreme ultraviolet (EUV), X-ray, and ion beam. The amount of the acid generating agent used is preferably 0 to 49% by mass of the total mass of the components except for the solvent (C), more preferably 0 to 40% by mass, still more preferably 0 to 30% by mass, and particularly preferably 0 to 25% by mass. By using the acid generating agent within the above range, a pattern profile with high sensitivity and low edge roughness is obtained. In the present embodiment, the acid generation method is not limited as long as an acid is generated in the system. By using excimer laser instead of ultraviolet such as g-ray and i-ray, finer processing is possible, and also by using electron beam, extreme ultraviolet, X-ray or ion beam as a high energy ray, further finer processing is possible.

The acid generating agent is preferably at least one kind selected from the group consisting of compounds represented by the following formulae (7-1) to (7-8): 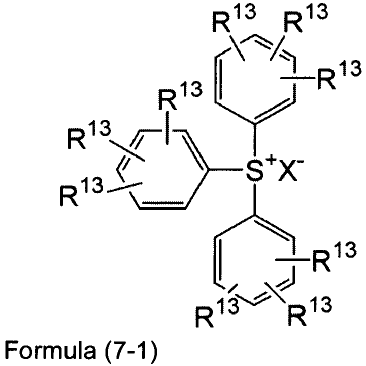 (In the formula (7-1), R¹³ may be the same or different, and are each independently a hydrogen atom, a linear, branched or cyclic alkyl group, a linear, branched or cyclic alkoxy group, a hydroxyl group, or a halogen atom; X⁻ is an alkyl group, an aryl group, a sulfonic acid ion having a halogen substituted alkyl group or a halogen substituted aryl group, or a halide ion.)

The compound represented by the above formula (7-1) is preferably at least one kind selected from the group consisting of triphenylsulfonium trifluoromethanesulfonate, triphenylsulfonium nonafluoro-n-butanesulfonate, diphenyltolylsulfonium nonafluoro-n-butanesulfonate, triphenylsulfonium perfluoro-n-octanesulfonate, diphenyl-4-methylphenylsulfonium trifluoromethanesulfonate, di-2,4,6-trimethylphenylsulfonium trifluoromethanesulfonate, diphenyl-4-t-butoxyphenylsulfonium trifluoromethanesulfonate, diphenyl-4-t-butoxyphenylsulfonium nonafluoro-n-butanesulfonate, diphenyl-4-hydroxyphenylsulfonium trifluoromethanesulfonate, bis(4-fluorophenyl)-4-hydroxyphenylsulfonium trifluoromethanesulfonate, diphenyl-4-hydroxyphenylsulfonium nonafluoro-n-butanesulfonate, bis(4-hydroxyphenyl)-phenylsulfonium trifluoromethanesulfonate, tri(4-methoxyphenyl)sulfonium trifluoromethanesulfonate, tri(4-fluorophenyl)sulfonium trifluoromethanesulfonate, triphenylsulfonium p-toluenesulfonate, triphenylsulfonium benzenesulfonate, diphenyl-2,4,6-trimethylphenyl-p-toluenesulfonate, diphenyl-2,4,6-trimethylphenylsulfonium-2-trifluoromethylbenzenesulfonate, diphenyl-2,4,6-trimethylphenylsulfonium-4-trifluoromethylbenzenesulfonate, diphenyl-2,4,6-trimethylphenylsulfonium-2,4-difluorobenzenesulfonate, diphenyl-2,4,6-trimethylphenylsulfonium hexafluorobenzenesulfonate, diphenylnaphthylsulfonium trifluoromethanesulfonate, diphenyl-4-hydroxyphenylsulfonium-p-toluenesulfonate, triphenylsulfonium 10-camphorsulfonate, diphenyl-4-hydroxyphenylsulfonium 10-camphorsulfonate, and cyclo(1,3-perfluoropropanedisulfone)imidate. 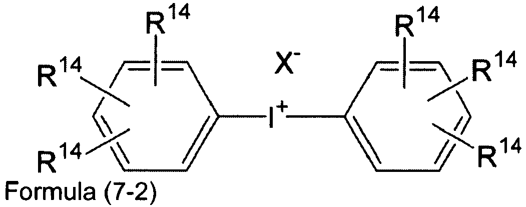 (In the formula (7-2), R¹⁴ may be the same or different, and each independently represents a hydrogen atom, a linear, branched or cyclic alkyl group, a linear, branched or cyclic alkoxy group, a hydroxyl group, or a halogen atom. X⁻ is the same as above.)

The compound represented by the above formula (7-2) is preferably at least one kind selected from the group consisting of bis(4-t-butylphenyl)iodonium trifluoromethanesulfonate, bis(4-t-butylphenyl)iodonium nonafluoro-n-butanesulfonate, bis(4-t-butylphenyl)iodonium perfluoro-n-octanesulfonate, bis(4-t-butylphenyl)iodonium p-toluenesulfonate, bis(4-t-butylphenyl)iodonium benzenesulfonate, bis(4-t-butylphenyl)iodonium-2-trifluoromethylbenzenesulfonate, bis(4-t-butylphenyl)iodonium-4-trifluoromethylbenzenesulfonate, bis(4-t-butylphenyl)iodonium-2,4-difluorobenzenesulfonate, bis(4-t-butylphenyl)iodonium hexafluorobenzenesulfonate, bis(4-t-butylphenyl)iodonium 10-camphorsulfonate, diphenyliodonium trifluoromethanesulfonate, diphenyliodonium nonafluoro-n-butanesulfonate, diphenyliodonium perfluoro-n-octanesulfonate, diphenyliodonium p-toluenesulfonate, diphenyliodonium benzenesulfonate, diphenyliodonium 10-camphorsulfonate, diphenyliodonium-2-trifluoromethylbenzenesulfonate, diphenyliodonium-4-trifluoromethylbenzenesulfonate, diphenyliodonium-2,4-difluorobenzenesulfonate, diphenyliodonium hexafluorobenzenesulfonate, di(4-trifluoromethylphenyl)iodonium trifluoromethanesulfonate, di(4-trifluoromethylphenyl)iodonium nonafluoro-n-butanesulfonate, di(4-trifluoromethylphenyl)iodonium perfluoro-n-octanesulfonate, di(4-trifluoromethylphenyl)iodonium p-toluenesulfonate, di(4-trifluoromethylphenyl)iodonium benzenesulfonate, and di(4-trifluoromethylphenyl)iodonium 10-camphersulfonate. 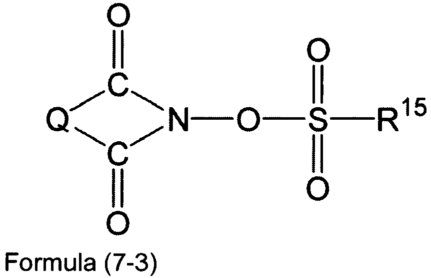 (In the formula (7-3), Q is an alkylene group, an arylene group, or an alkoxylene group, and R¹⁵ is an alkyl group, an aryl group, a halogen substituted alkyl group, or a halogen substituted aryl group.)

The compound represented by the above formula (7-3) is preferably at least one kind selected from the group consisting of N-(trifluoromethylsulfonyloxy)succinimide, N-(trifluoromethylsulfonyloxy)phthalimide, N-(trifluoromethylsulfonyloxy)diphenylmaleimide, N-(trifluoromethylsulfonyloxy)bicyclo[2.2.1]hept-5-en-2,3-dicarboxyimide, N-(trifluoromethylsulfonyloxy)naphthylimide, N-(10-camphorsulfonyloxy)succinimide, N-(10-camphorsulfonyloxy)phthalimide, N-(10-camphorsulfonyloxy)diphenylmaleimide, N-(10-camphorsulfonyloxy)bicyclo[2.2.1]hept-5-en-2,3-dicarboxyimide, N-(10-camphorsulfonyloxy)naphthylimide, N-(n-octanesulfonyloxy)bicyclo[2.2.1]hept-5-en-2,3-dicarboxyimide, N-(n-octanesulfonyloxy)naphthylimide, N-(p-toluenesulfonyloxy)bicyclo[2.2.1]hept-5-en-2,3-dicarboxyimide, N-(p-toluenesulfonyloxy)naphthylimide, N-(2-trifluoromethylbenzenesulfonyloxy)bicyclo[2.2.1] hept-5-en-2,3-dicarboxyimide, N-(2-trifluoromethylbenzenesulfonyloxy)naphthylimide, N-(4-trifluoromethylbenzenesulfonyloxy)bicyclo[2.2.1]hept-5-en-2,3-dicarboxyimide, N-(4-trifluoromethylbenzenesulfonyloxy)naphthylimide, N-(perfluorobenzenesulfonyloxy)bicyclo[2.2.1]hept-5-en-2,3-dicarboxyimide, N-(perfluorobenzenesulfonyloxy)naphthylimide, N-(1-naphthalenesulfonyloxy)bicyclo[2.2.1]hept-5-en-2,3-dicarboxyimide, N-(1-naphthalenesulfonyloxy)naphthylimide, N-(nonafluoro-n-butanesulfonyloxy)bicyclo[2.2.1]hept-5-en-2,3-dicarboxyimide, N-(nonafluoro-n-butanesulfonyloxy)naphthylimide, N-(perfluoro-n-octanesulfonyloxy)bicyclo[2.2.1]hept-5-en-2,3-dicarboxyimide, and N-(perfluoro-n-octanesulfonyloxy)naphthylimide. 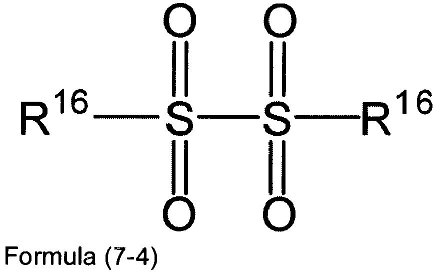 (In the formula (7-4), R¹⁶ may be the same or different, and are each independently an optionally substituted linear, branched or cyclic alkyl group, an optionally substituted aryl group, an optionally substituted heteroaryl group, or an optionally substituted aralkyl group.)

The compound represented by the above formula (7-4) is preferably at least one kind selected from the group consisting of diphenyldisulfone, di(4-methylphenyl)disulfone, dinaphthyldisulfone, di(4-tert-butylphenyl)disulfone, di(4-hydroxyphenyl)disulfone, di(3-hydroxynaphthyl)disulfone, di(4-fluorophenyl)disulfone, di(2-fluorophenyl)disulfone, and di(4-trifluoromethylphenyl)disulfone. 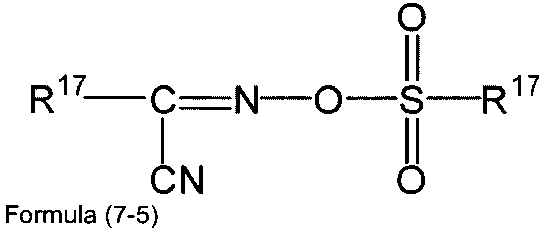 (In the formula (7-5), R¹⁷ may be the same or different, and are each independently an optionally substituted linear, branched or cyclic alkyl group, an optionally substituted aryl group, an optionally substituted heteroaryl group, or an optionally substituted aralkyl group.)

The compound represented by the above formula (7-5) is preferably at least one kind selected from the group consisting of α-(methylsulfonyloxyimino)-phenylacetonitrile, α-(methylsulfonyloxyimino)-4-methoxyphenylacetonitrile, α-(trifluoromethylsulfonyloxyimino)-phenylacetonitrile, α-(trifluoromethylsulfonyloxyimino)-4-methoxyphenylacetonitrile, α-(ethylsulfonyloxyimino)-4-methoxyphenylacetonitrile, α-(propylsulfonyloxyimino)-4-methylphenylacetonitrile, and α-(methylsulfonyloxyimino)-4-bromophenylacetonitrile.

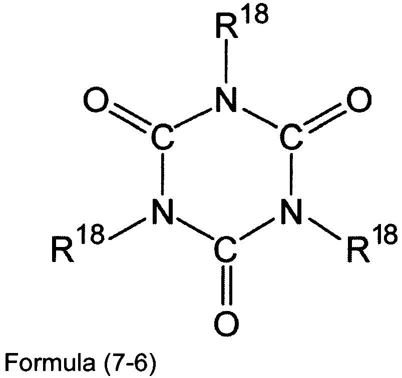

In the formula (7-6), R¹⁸ may be the same or different, and are each independently a halogenated alkyl group having one or more chlorine atoms and one or more bromine atoms. The number of carbon atoms in the halogenated alkyl group is preferably 1 to 5.

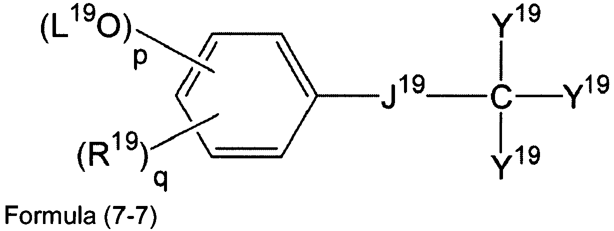

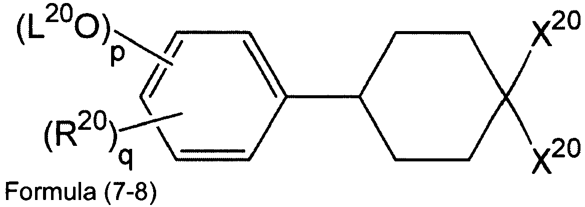

In the formulae (7-7) and (7-8), R¹⁹ and R²⁰ are each independently an alkyl group of 1 to 3 carbon atoms such as a methyl group, an ethyl group, an n-propyl group, and an isopropyl group; a cycloalkyl group such as a cyclopentyl group and a cyclohexyl group; an alkoxyl group of 1 to 3 carbon atoms such as a methoxy group, an ethoxy group, and a propoxy group; or an aryl group such as a phenyl group, a toluoyl group, and a naphthyl group, and preferably an aryl group of 6 to 10 carbon atoms. L¹⁹ and L²⁰ are each independently an organic group having a 1,2-naphthoquinonediazide group. Specifically, preferable examples of the organic group having a 1,2-naphthoquinonediazide group include a 1,2-quinonediazidesulfonyl group such as a 1,2-naphthoquinonediazide-4-sulfonyl group, a 1,2-naphthoquinonediazide-5-sulfonyl group, and a 1,2-naphthoquinonediazide-6-sulfonyl group. A 1,2-naphthoquinonediazide-4-sulfonyl group and a 1,2-naphthoquinonediazide-5-sulfonyl group are particularly preferable. p is an integer of 1 to 3; q is an integer of 0 to 4; and 1≤ p+q≤5. J¹⁹ is a single bond, a polymethylene group of 1 to 4 carbon atoms, a cycloalkylene group, a phenylene group, a group represented by the following formula (7-7-1), a carbonyl group, an ester group, an amide group, or an ether group. Y¹⁹ is a hydrogen atom, an alkyl group, or an aryl group, and X²⁰ are each independently a group represented by the following formula (7-8-1): 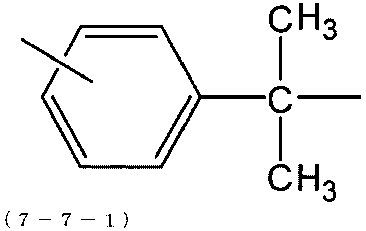 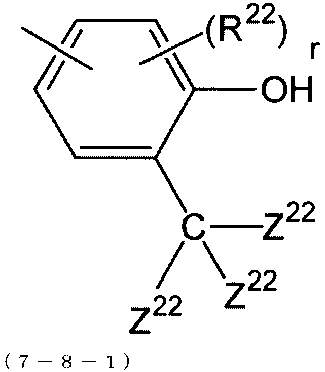 (In the above formula (7-8-1), Z²² are each independently an alkyl group, a cycloalkyl group, or an aryl group; R²² is an alkyl group, a cycloalkyl group, or an alkoxyl group; and r is an integer of 0 to 3.)

Examples of the other acid generating agent include bissulfonyldiazomethanes such as bis(p-toluenesulfonyl)diazomethane, bis(2,4-dimethylphenylsulfonyl)diazomethane, bis(tert-butylsulfonyl)diazomethane, bis(n-butylsulfonyl)diazomethane, bis(isobutylsulfonyl)diazomethane, bis(isopropylsulfonyl)diazomethane, bis(n-propylsulfonyl)diazomethane, bis(cyclohexylsulfonyl)diazomethane, bis(isopropylsulfonyl)diazomethane, 1,3-bis(cyclohexylsulfonylazomethylsulfonyl)propane, 1,4-bis(phenylsulfonylazomethylsulfonyl)butane, 1,6-bis(phenylsulfonylazomethylsulfonyl)hexane, and 1,10-bis(cyclohexylsulfonylazomethylsulfonyl)decane; and halogen-containing triazine derivatives such as 2-(4-methoxyphenyl)-4,6-(bistrichloromethyl)-1,3,5-triazine, 2-(4-methoxynaphthyl)-4,6-(bistrichloromethyl)-1,3,5-triazine, tris(2,3-dibromopropyl)-1,3,5-triazine, and tris(2,3-dibromopropyl)isocyanurate.

Among the above acid generating agents, an acid generating agent having an aromatic ring is preferable, and an acid generating agent represented by the formula (7-1) or (7-2) is more preferable. An acid generating agent having a sulfonate ion wherein X⁻ of the formula (7-1) or (7-2) has an aryl group or a halogen-substituted aryl group is still more preferable; an acid generating agent having a sulfonate ion wherein X⁻ of the formula (7-1) or (7-2) has an aryl group is particularly preferable; and diphenyltrimethylphenylsulfonium p-toluenesulfonate, triphenylsulfonium p-toluenesulfonate, triphenylsulfonium trifluoromethanesulfonate, and triphenylsulfonium nonafluoromethanesulfonate are particularly preferable. By using the acid generating agent, LER can be reduced.

The acid generating agent can be used alone or in combination of two or more kinds.

### (2) Acid Crosslinking Agent

The radiation-sensitive composition of the present embodiment may contain one or more acid crosslinking agents. The acid crosslinking agent is a compound capable of intramolecular or intermolecular crosslinking the resist base material represented by the formula (1) in the presence of the acid generated from the acid generating agent. Examples of such an acid crosslinking agent include a compound having one or more groups (hereinafter, referred to as "crosslinkable group") capable of crosslinking the resist base material of the formula (1).

Specific examples of such a crosslinkable group include (i) a hydroxyalkyl group such as a hydroxy (C1-C6 alkyl group), a C1-C6 alkoxy (C1-C6 alkyl group), and an acetoxy (C1-C6 alkyl group), or a group derived therefrom; (ii) a carbonyl group such as a formyl group and a carboxy (C1-C6 alkyl group), or a group derived therefrom; (iii) a nitrogenous group-containing group such as a dimethylaminomethyl group, a diethylaminomethyl group, a dimethylolaminomethyl group, a diethylolaminomethyl group, and a morpholinomethyl group; (iv) a glycidyl group-containing group such as a glycidyl ether group, a glycidyl ester group, and a glycidylamino group; (v) a group derived from an aromatic group such as a C1-C6 allyloxy (C1-C6 alkyl group) and a C1-C6 aralkyloxy (C1-C6 alkyl group) such as a benzyloxymethyl group and a benzoyloxymethyl group; and (vi) a polymerizable multiple bond-containing group such as a vinyl group and an isopropenyl group. As the crosslinkable group of the acid crosslinking agent of the present invention, a hydroxyalkyl group and an alkoxyalkyl group or the like are preferable, and an alkoxymethyl group is particularly preferable.

Examples of the acid crosslinking agent having the above crosslinkable group include (i) a methylol group-containing compound such as a methylol group-containing melamine compound, a methylol group-containing benzoguanamine compound, a methylol group-containing urea compound, a methylol group-containing glycoluryl compound, and a methylol group-containing phenolic compound; (ii) an alkoxyalkyl group-containing compound such as an alkoxyalkyl group-containing melamine compound, an alkoxyalkyl group-containing benzoguanamine compound, an alkoxyalkyl group-containing urea compound, an alkoxyalkyl group-containing glycoluryl compound, and an alkoxyalkyl group-containing phenolic compound; (iii) a carboxymethyl group-containing compound such as a carboxymethyl group-containing melamine compound, a carboxymethyl group-containing benzoguanamine compound, a carboxymethyl group-containing urea compound, a carboxymethyl group-containing glycoluryl compound, and a carboxymethyl group-containing phenolic compound; (iv) an epoxy compound such as a bisphenol A based epoxy compound, a bisphenol F based epoxy compound, a bisphenol S based epoxy compound, a novolac resin based epoxy compound, a resol resin based epoxy compound, and a poly(hydroxystyrene) based epoxy compound.

As the acid crosslinking agent, a compound having a phenolic hydroxyl group, and a compound and resin where the above crosslinkable group is introduced into an acid functional group in an alkali soluble resin to impart crosslinkability can be further used. The introduction rate of the crosslinkable group in that case is adjusted to be usually 5 to 100 mol %, preferably 10 to 60 mol %, and more preferably 15 to 40 mol % based on the total acid functional groups in the compound having a phenolic hydroxyl group, and the alkali soluble resin. Within the above range, the crosslinking reaction sufficiently occurs, and a decrease in the film remaining rate, and swelling phenomena and meandering or the like of a pattern are avoided, which is preferable.

In the radiation-sensitive composition of the present embodiment, the acid crosslinking agent is preferably an alkoxyalkylated urea compound or resin thereof, or an alkoxyalkylated glycoluryl compound or resin thereof. Particularly preferable examples of the acid crosslinking agent include compounds represented by the following formulae (8-1) to (8-3) and an alkoxymethylated melamine compound (acid crosslinking agent). 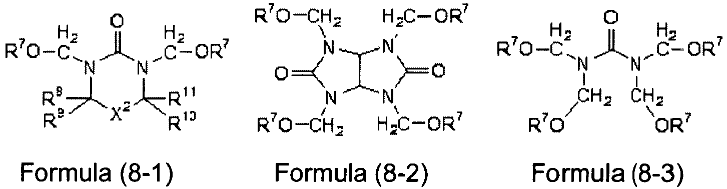 (In the above formulae (8-1) to (8-3), R⁷ each independently represents a hydrogen atom, an alkyl group, or an acyl group; R⁸ to R¹¹ each independently represents a hydrogen atom, a hydroxyl group, an alkyl group, or an alkoxyl group; and X² represents a single bond, a methylene group, or an oxygen atom.)

The alkyl group represented by R⁷ is preferably 1 to 6 carbon atoms, and more preferably 1 to 3 carbon atoms. Examples thereof include a methyl group, an ethyl group, and a propyl group. The acyl group represented by R⁷ is preferably 2 to 6 carbon atoms, and more preferably 2 to 4 carbon atoms. Examples thereof include an acetyl group and a propionyl group. The alkyl group represented by R⁸ to R¹¹ is preferably 1 to 6 carbon atoms, and more preferably 1 to 3 carbon atoms. Examples thereof include a methyl group, an ethyl group, and a propyl group. The alkoxy group represented by R⁸ to R¹¹ is preferably 1 to 6 carbon atoms, and more preferably 1 to 3 carbon atoms. Examples thereof include a methoxy group, an ethoxy group, and a propoxy group. X² is preferably a single bond or a methylene group. R⁷ to R¹¹ and X² may be substituted with an alkyl group such as a methyl group and an ethyl group, an alkoxy group such as a methoxy group and an ethoxy group, a hydroxyl group, and a halogen atom or the like. A plurality of R⁷ and R⁸ to R¹¹ may be each the same or different.

Specific examples of the compound represented by the formula (8-1) can include compounds shown below:

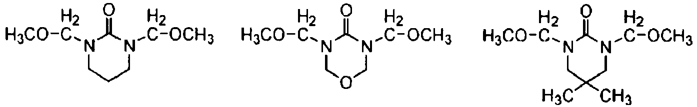

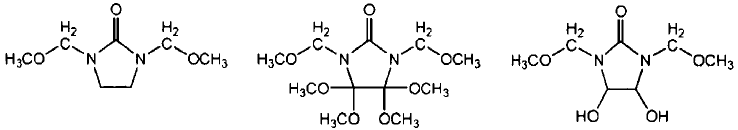

Specific examples of the compound represented by the formula (8-2) include N,N,N,N-tetra(methoxymethyl)glycoluryl, N,N,N,N-tetra(ethoxymethyl)glycoluryl, N,N,N,N-tetra(n-propoxymethyl)glycoluryl, N,N,N,N-tetra(isopropoxymethyl)glycoluryl, N,N,N,N-tetra(n-butoxymethyl)glycoluryl, and N,N,N,N-tetra(t-butoxymethyl)glycoluryl. Among these, N,N,N,N-tetra(methoxymethyl)glycoluryl is particularly preferable.

Specific examples of the compound represented by the formula (8-3) include compounds represented below:

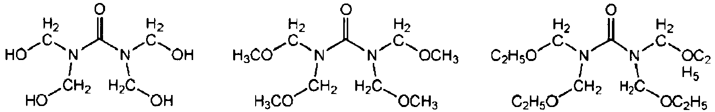

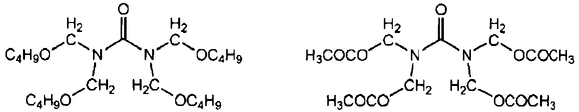

Specific examples of the alkoxymethylated melamine compound include N,N,N,N,N,N-hexa(methoxymethyl)melamine, N,N,N,N,N,N-hexa(ethoxymethyl)melamine, N,N,N,N,N,N-hexa(n-propoxymethyl)melamine, N,N,N,N,N,N-hexa(isopropoxymethyl)melamine, N,N,N,N,N,N-hexa(n-butoxymethyl)melamine, and N,N,N,N,N,N-hexa(t-butoxymethyl)melamine. Among these, N,N,N,N,N,N-hexa(methoxymethyl)melamine is particularly preferable.

The above acid crosslinking agent can be obtained by, for example, conducting a condensation reaction of a urea compound or a glycoluryl compound with formalin to introduce a methylol group, etherifying the product with lower alcohols such as methyl alcohol, ethyl alcohol, propyl alcohol, and butyl alcohol, and then cooling the reaction solution to collect a precipitated compound or resin thereof. The above acid crosslinking agent can be obtained as a commercially available product such as CYMEL (trade name, manufactured by MT AquaPolymer) and NIKALAC (manufactured by Sanwa Chemical).

Other particularly preferable examples of the acid crosslinking agent include a phenol derivative having 1 to 6 benzene rings within a molecule and two or more hydroxyalkyl groups and/or alkoxyalkyl groups within the entire molecule, the hydroxyalkyl groups and/or alkoxyalkyl groups being bonded to any of the above benzene rings. Preferable examples thereof include a phenol derivative having a molecular weight of 1500 or less, 1 to 6 benzene rings and a total of two or more hydroxyalkyl groups and/or alkoxyalkyl groups within a molecule, the hydroxyalkyl groups and/or alkoxyalkyl groups being bonded to any one of the above benzene rings, or a plurality of benzene rings.

The hydroxyalkyl group bonded to a benzene ring is the one of 1 to 6 carbon atoms such as a hydroxymethyl group, a 2-hydroxyethyl group, and a 2-hydroxy-1-propyl group is preferable. As the alkoxyalkyl group bonded to a benzene ring, the one of 2 to 6 carbon atoms is preferable. Specifically, a methoxymethyl group, an ethoxymethyl group, an n-propoxymethyl group, an isopropoxymethyl group, an n-butoxymethyl group, an isobutoxymethyl group, a sec-butoxymethyl group, a t-butoxymethyl group, a 2-methoxyethyl group, or a 2-methoxy-1-propyl group is preferable.

Among these phenol derivatives, particularly preferable ones are shown below:

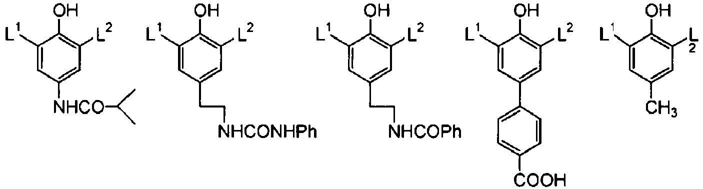

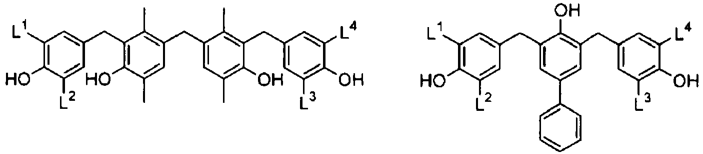

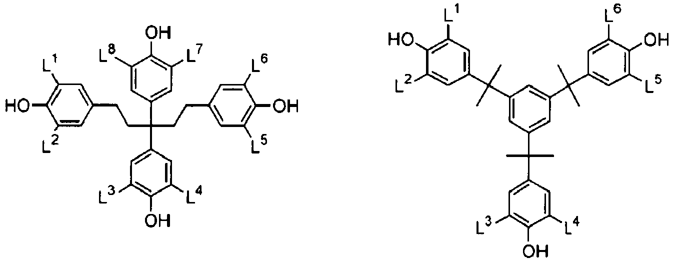

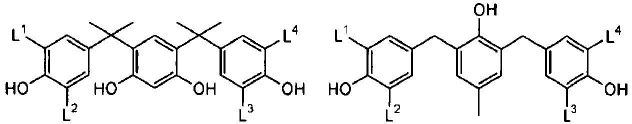

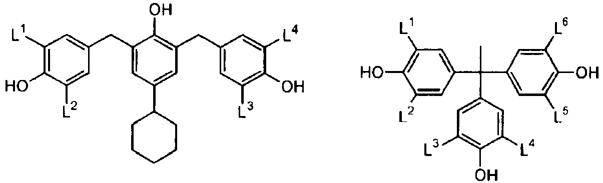

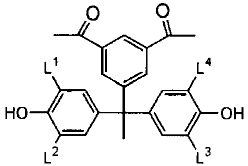

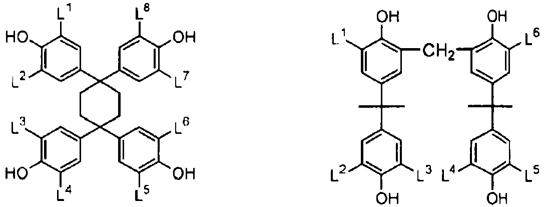

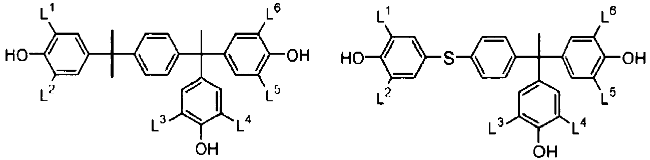

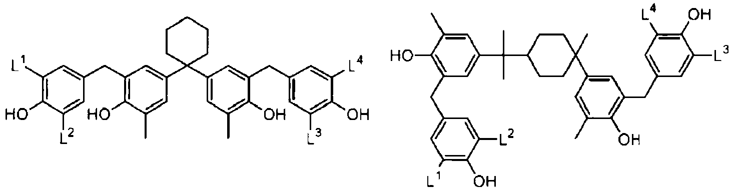

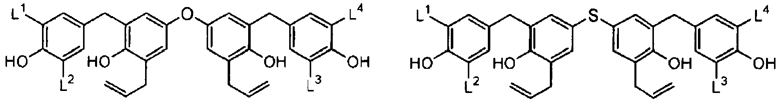

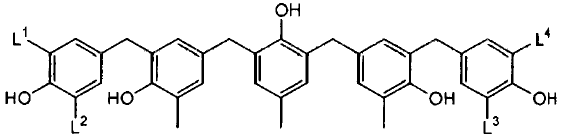

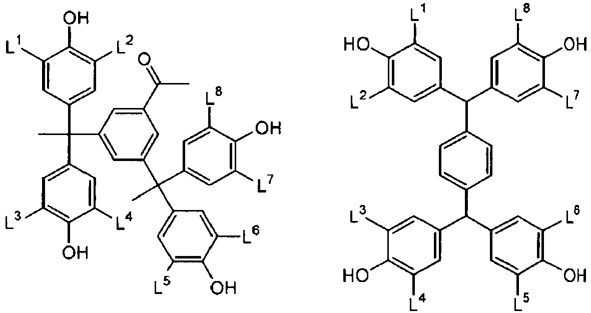

In the above formulae, L¹ to L⁸ may be the same or different, and each independently represents a hydroxymethyl group, a methoxymethyl group, or an ethoxymethyl group. A phenol derivative having a hydroxymethyl group can be obtained by reacting the corresponding phenolic compound having no hydroxymethyl group (a compound where L¹ to L⁸ in the above formulae are a hydrogen atom) with formaldehyde in the presence of a basic catalyst. In this case, in order to prevent resinification and gelation, the reaction temperature is preferably 60°C or less. Specifically, it can be synthesized by methods described in Japanese Patent Application Laid-Open Nos. 6-282067 and 7-64285 or the like.

A phenol derivative having an alkoxymethyl group can be obtained by reacting the corresponding phenol derivative having a hydroxymethyl group with an alcohol in the presence of an acid catalyst. In this case, in order to prevent resinification and gelation, the reaction temperature is preferably 100°C or less. Specifically, it can be synthesized by methods described in EP632003A1 or the like.

While the phenol derivative having a hydroxymethyl group and/or an alkoxymethyl group thus synthesized is preferable in terms of stability upon storage, the phenol derivative having an alkoxymethyl group is particularly preferable in terms of stability upon storage. The acid crosslinking agent may be used alone, or may be used in combination of two or more kinds.

Other particularly preferable examples of the acid crosslinking agent include a compound having at least one α-hydroxyisopropyl group. The compound is not particularly limited in the structure, as long as it has an α-hydroxyisopropyl group. A hydrogen atom of a hydroxyl group in the above α-hydroxyisopropyl group may be substituted with one or more acid dissociation groups (R-COO- group, R-SO₂- group or the like, wherein R represents a substituent group selected from the group consisting of a linear hydrocarbon group of 1 to 12 carbon atoms, a cyclic hydrocarbon group of 3 to 12 carbon atoms, an alkoxy group of 1 to 12 carbon atoms, a 1-branched alkyl group of 3 to 12 carbon atoms, and an aromatic hydrocarbon group of 6 to 12 carbon atoms). Examples of a compound having the above α-hydroxyisopropyl group include one kind or two kinds or more of a substituted or non-substituted aromatic based compound, a diphenyl compound, a naphthalene compound, a furan compound or the like containing at least one α-hydroxyisopropyl group. Specific examples thereof include a compound represented by the following general formula (9-1) (hereinafter, referred to as "benzene based compound (1)"), a compound represented by the following general formula (9-2) (hereinafter, referred to as "diphenyl based compound (2)"), a compound represented by the following general formula (9-3) (hereinafter, referred to as "naphthalene based compound (3)"), and a compound represented by the following general formula (9-4) (hereinafter, referred to as "furan based compound (4)").

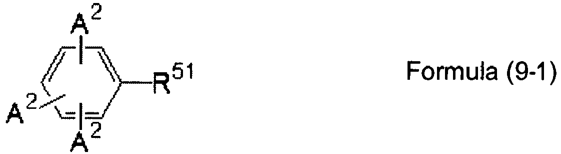

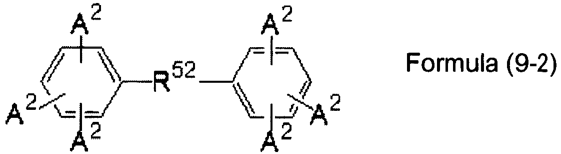

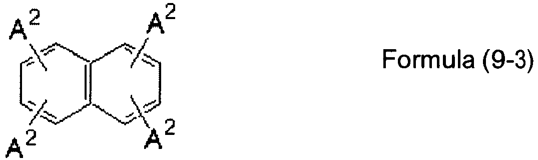

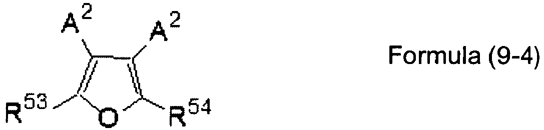

In the above general formulae (9-1) to (9-4), each A² independently represents an α-hydroxyisopropyl group or a hydrogen atom, and at least one A² is an α-hydroxyisopropyl group. In the general formula (9-1), R⁵¹ represents a hydrogen atom, a hydroxyl group, a linear or branched alkylcarbonyl group of 2 to 6 carbon atoms, or a linear or branched alkoxycarbonyl group of 2 to 6 carbon atoms. Furthermore, in the general formula (9-2), R⁵² represents a single bond, a linear or branched alkylene group of 1 to 5 carbon atoms, -O-, -CO-, or -COO-. Also, in the general formula (9-4), R⁵³ and R⁵⁴ represent a hydrogen atom or a linear or branched alkyl group of 1 to 6 carbon atoms independently from each other.

Specific examples of the benzene based compound (1) include α-hydroxyisopropylbenzenes such as α-hydroxyisopropylbenzene, 1,3-bis(α-hydroxyisopropyl)benzene, 1,4-bis(α-hydroxyisopropyl)benzene, 1,2,4-tris(α-hydroxyisopropyl)benzene, and 1,3,5-tris(α-hydroxyisopropyl)benzene; α-hydroxyisopropylphenols such as 3-α-hydroxyisopropylphenol, 4-α-hydroxyisopropylphenol, 3,5-bis(α-hydroxyisopropyl)phenol, and 2,4,6-tris(α-hydroxyisopropyl)phenol; α-hydroxyisopropylphenyl alkyl ketones such as 3-α-hydroxyisopropylphenyl methyl ketone, 4-α-hydroxyisopropylphenyl methyl ketone, 4-α-hydroxyisopropylphenyl ethyl ketone, 4-α-hydroxyisopropylphenyl-n-propyl ketone, 4-α-hydroxyisopropylphenyl isopropyl ketone, 4-α-hydroxyisopropylphenyl-n-butyl ketone, 4-α-hydroxyisopropylphenyl-t-butyl ketone, 4-α-hydroxyisopropylphenyl-n-pentyl ketone, 3,5-bis(α-hydroxyisopropyl)phenyl methyl ketone, 3,5-bis(α-hydroxyisopropyl)phenyl ethyl ketone, and 2,4,6-tris(α-hydroxyisopropyl)phenyl methyl ketone; alkyl 4-α-hydroxyisopropylbenzoates such as methyl 3-α-hydroxyisopropylbenzoate, methyl 4-α-hydroxyisopropylbenzoate, ethyl 4-α-hydroxyisopropylbenzoate, n-propyl 4-α-hydroxyisopropylbenzoate, isopropyl 4-α-hydroxyisopropylbenzoate, n-butyl 4-α-hydroxyisopropylbenzoate, t-butyl 4-α-hydroxyisopropylbenzoate, n-pentyl 4-α-hydroxyisopropylbenzoate, methyl 3,5-bis(α-hydroxyisopropyl)benzoate, ethyl 3,5-bis(α-hydroxyisopropyl)benzoate, and methyl 2,4,6-tris(α-hydroxyisopropyl)benzoate.

Specific examples of the above diphenyl based compound (2) include α-hydroxyisopropylbiphenyls such as 3-α-hydroxyisopropylbiphenyl, 4-α-hydroxyisopropylbiphenyl, 3,5-bis(α-hydroxyisopropyl)biphenyl, 3,3'-bis(α-hydroxyisopropyl)biphenyl, 3,4'-bis(α-hydroxyisopropyl)biphenyl, 4,4'-bis(α-hydroxyisopropyl)biphenyl, 2,4,6-tris(α-hydroxyisopropyl)biphenyl, 3,3',5-tris(α-hydroxyisopropyl)biphenyl, 3,4',5-tris(α-hydroxyisopropyl)biphenyl, 2,3',4,6,-tetrakis(α-hydroxyisopropyl)biphenyl, 2,4,4',6,-tetrakis(α-hydroxyisopropyl)biphenyl, 3,3',5,5'-tetrakis(α-hydroxyisopropyl)biphenyl, 2,3',4,5',6-pentakis(α-hydroxyisopropyl)biphenyl, and 2,2',4,4',6,6'-hexakis(α-hydroxyisopropyl)biphenyl; α-hydroxyisopropyldiphenylalkanes such as 3-α-hydroxyisopropyldiphenylmethane, 4-α-hydroxyisopropyldiphenylmethane, 1-(4-α-hydroxyisopropylphenyl)-2-phenylethane, 1-(4-α-hydroxyisopropylphenyl)-2-phenylpropane, 2-(4-α-hydroxyisopropylphenyl)-2-phenylpropane, 1-(4-α-hydroxyisopropylphenyl)-3-phenylpropane, 1-(4-α-hydroxyisopropylphenyl)-4-phenylbutane, 1-(4-α-hydroxyisopropylphenyl)-5-phenylpentane, 3,5-bis(α-hydroxyisopropyldiphenylmethane, 3,3'-bis(α-hydroxyisopropyl)diphenylmethane, 3,4'-bis(α-hydroxyisopropyl)diphenylmethane, 4,4'-bis(α-hydroxyisopropyl)diphenylmethane, 1,2-bis(4-α-hydroxyisopropylphenyl)ethane, 1,2-bis(4-α-hydroxypropylphenyl)propane, 2,2-bis(4-α-hydroxypropylphenyl)propane, 1,3-bis(4-α-hydroxypropylphenyl)propane, 2,4,6-tris(α-hydroxyisopropyl)diphenylmethane, 3,3',5-tris(α-hydroxyisopropyl)diphenylmethane, 3,4',5-tris(α-hydroxyisopropyl)diphenylmethane, 2,3',4,6-tetrakis(α-hydroxyisopropyl)diphenylmethane, 2,4,4',6-tetrakis(α-hydroxyisopropyl)diphenylmethane, 3,3',5,5'-tetrakis(α-hydroxyisopropyl)diphenylmethane, 2,3',4,5',6-pentakis(α-hydroxyisopropyl)diphenylmethane, and 2,2',4,4',6,6'-hexakis(α-hydroxyisopropyl)diphenylmethane; α-hydroxyisopropyldiphenyl ethers such as 3-α-hydroxyisopropyldiphenyl ether, 4-α-hydroxyisopropyldiphenyl ether, 3,5-bis(α-hydroxyisopropyl)diphenyl ether, 3,3'-bis(α-hydroxyisopropyl)diphenyl ether, 3,4'-bis(α-hydroxyisopropyl)diphenyl ether, 4,4'-bis(α-hydroxyisopropyl)diphenyl ether, 2,4,6-tris(α-hydroxyisopropyl)diphenyl ether, 3,3',5-tris(α-hydroxyisopropyl)diphenyl ether, 3,4',5-tris(α-hydroxyisopropyl)diphenyl ether, 2,3',4,6-tetrakis(α-hydroxyisopropyl)diphenyl ether, 2,4,4',6-tetrakis(α-hydroxyisopropyl)diphenyl ether, 3,3',5,5'-tetrakis(α-hydroxyisopropyl)diphenyl ether, 2,3',4,5',6-pentakis(α-hydroxyisopropyl)diphenyl ether, and 2,2',4,4',6,6'-hexakis(α-hydroxyisopropyl)diphenyl ether; α-hydroxyisopropyldiphenyl ketones such as 3-α-hydroxyisopropyldiphenyl ketone, 4-α-hydroxyisopropyldiphenyl ketone, 3,5-bis(α-hydroxyisopropyl)diphenyl ketone, 3,3'-bis(α-hydroxyisopropyl)diphenyl ketone, 3,4'-bis(α-hydroxyisopropyl)diphenyl ketone, 4,4'-bis(α-hydroxyisopropyl)diphenyl ketone, 2,4,6-tris(α-hydroxyisopropyl)diphenyl ketone, 3,3',5-tris(α-hydroxyisopropyl)diphenyl ketone, 3,4',5-tris(α-hydroxyisopropyl)diphenyl ketone, 2,3',4,6-tetrakis(α-hydroxyisopropyl)diphenyl ketone, 2,4,4',6-tetrakis(α-hydroxyisopropyl)diphenyl ketone, 3,3',5,5'-tetrakis(α-hydroxyisopropyl)diphenyl ketone, 2,3',4,5',6-pentakis(α-hydroxyisopropyl)diphenyl ketone, and 2,2',4,4',6,6'-hexakis(α-hydroxyisopropyl)diphenyl ketone; phenyl α-hydroxyisopropylbenzoates such as phenyl 3-α-hydroxyisopropylbenzoate, phenyl 4-α-hydroxyisopropylbenzoate, 3-α-hydroxyisopropylphenyl benzoate, 4-α-hydroxyisopropylphenyl benzoate, phenyl 3,5-bis(α-hydroxyisopropyl)benzoate, 3-α-hydroxyisopropylphenyl 3-α-hydroxyisopropylbenzoate, 4-α-hydroxyisopropylphenyl 3-α-hydroxyisopropylbenzoate, 3-α-hydroxyisopropylphenyl 4-α-hydroxyisopropylbenzoate, 4-α-hydroxyisopropylphenyl 4-α-hydroxyisopropylbenzoate, 3,5-bis(α-hydroxyisopropyl)phenyl benzoate, phenyl 2,4,6-tris(α-hydroxyisopropyl)benzoate, 3-α-hydroxyisopropylphenyl 3,5-bis(α-hydroxyisopropyl)benzoate, 4-α-hydroxyisopropylphenyl 3,5-bis(α-hydroxyisopropyl)benzoate, 3,5-bis(α-hydroxyisopropyl)phenyl 3-α-hydroxyisopropylbenzoate, 3,5-bis(α-hydroxyisopropyl)phenyl 4-α-hydroxyisopropylbenzoate, 2,4,6-tris(α-hydroxyisopropyl)phenyl benzoate, 3-α-hydroxyisopropylphenyl 2,4,6-tris(α-hydroxyisopropyl)benzoate, 4-α-hydroxyisopropylphenyl 2,4,6-tris(α-hydroxyisopropyl)benzoate, 3,5-bis(α-hydroxyisopropyl)phenyl 3,5-bis(α-hydroxyisopropyl)benzoate, 2,4,6-tris(α-hydroxyisopropyl)phenyl 3-α-hydroxyisopropylbenzoate, 2,4,6-tris(α-hydroxyisopropyl)phenyl 4-α-hydroxyisopropylbenzoate, 3,5-bis(α-hydroxyisopropyl)phenyl 2,4,6-tris(α-hydroxyisopropyl)benzoate, 2,4,6-tris(α-hydroxyisopropyl)phenyl 3,5-bis(α-hydroxyisopropyl)benzoate, and 2,4,6-tris(α-hydroxyisopropyl)phenyl 2,4,6-tris(α-hydroxyisopropyl)benzoate.

Furthermore, specific examples of the above naphthalene based compound (3) include 1-(α-hydroxyisopropyl)naphthalene, 2-(α-hydroxyisopropyl)naphthalene, 1,3-bis(α-hydroxyisopropyl)naphthalene, 1,4-bis(α-hydroxyisopropyl)naphthalene, 1,5-bis(α-hydroxyisopropyl)naphthalene, 1,6-bis(α-hydroxyisopropyl)naphthalene, 1,7-bis(α-hydroxyisopropyl)naphthalene, 2,6-bis(α-hydroxyisopropyl)naphthalene, 2,7-bis(α-hydroxyisopropyl)naphthalene, 1,3,5-tris(α-hydroxyisopropyl)naphthalene, 1,3,6-tris(α-hydroxyisopropyl)naphthalene, 1,3,7-tris(α-hydroxyisopropyl)naphthalene, 1,4,6-tris(α-hydroxyisopropyl)naphthalene, 1,4,7-tris(α-hydroxyisopropyl)naphthalene, and 1,3,5,7-tetrakis(α-hydroxyisopropyl)naphthalene.

Specific examples of the above furan based compound (4) include 3-(α-hydroxyisopropyl)furan, 2-methyl-3-(α-hydroxyisopropyl)furan, 2-methyl-4-(α-hydroxyisopropyl)furan, 2-ethyl-4-(α-hydroxyisopropyl)furan, 2-n-propyl-4-(α-hydroxyisopropyl)furan, 2-isopropyl-4-(α-hydroxyisopropyl)furan, 2-n-butyl-4-(α-hydroxyisopropyl)furan, 2-t-butyl-4-(α-hydroxyisopropyl)furan, 2-n-pentyl-4-(α-hydroxyisopropyl)furan, 2,5-dimethyl-3-(α-hydroxyisopropyl)furan, 2,5-diethyl-3-(α-hydroxyisopropyl)furan, 3,4-bis(α-hydroxyisopropyl)furan, 2,5-dimethyl-3,4-bis(α-hydroxyisopropyl)furan, and 2,5-diethyl-3,4-bis(α-hydroxyisopropyl)furan.

As the above acid crosslinking agent, a compound having two or more free α-hydroxyisopropyl groups is preferable; the above benzene based compound (1) having two or more α-hydroxyisopropyl groups, the above diphenyl based compound (2) having two or more α-hydroxyisopropyl groups, and the above naphthalene based compound (3) having two or more α-hydroxyisopropyl groups are more preferable; and α-hydroxyisopropylbiphenyls having two or more α-hydroxyisopropyl groups and the above naphthalene based compound (3) having two or more α-hydroxyisopropyl groups are particularly preferable.

The above acid crosslinking agent can normally be obtained by a method for reacting an acetyl group-containing compound such as 1,3-diacetylbenzene with Grignard reagent such as CH₃MgBr to methylate and then hydrolyzing, or a method for oxidizing an isopropyl group-containing compound such as 1,3-diisopropylbenzene with oxygen or the like to produce a peroxide and then reducing.

The amount of the acid crosslinking agent used in the present embodiment is preferably 0 to 49% by mass of the total mass of the components except for the solvent (C), more preferably 0 to 40% by mass, still more preferably 0 to 30% by mass, and particularly preferably 0 to 20% by mass. When the content of the above acid crosslinking agent exceeds 0% by mass, the inhibiting effect of the solubility of a resist film in an alkaline developing solution tends to be improved, and a decrease in the film remaining rate, and occurrence of swelling and meandering of a pattern tends to be able to be inhibited, which is preferable. On the other hand, when the content is 49% by mass or less, a decrease in heat resistance as a resist tends to be able to be inhibited, which is preferable.

The content of at least one kind of compound in the above acid crosslinking agent is also not particularly limited, and can be within various ranges according to the kind of substrates or the like used upon forming a resist pattern.

In all acid crosslinking agent components, preferably, the content of the alkoxymethylated melamine compound and/or the compounds represented by formulae (9-1) to (9-3) is 50 to 99% by mass, preferably 60 to 99% by mass, more preferably 70 to 98% by mass, and still more preferably 80 to 97% by mass. By having the alkoxymethylated melamine compound and/or the compounds represented by formulae (9-1) to (9-3) of 50% by mass or more of all acid crosslinking agent components, the resolution can be improved, which is preferable. By having the compounds of 99% by mass or less, the pattern cross section is more likely to have a rectangular shape, which is preferable.

### (3) Acid Diffusion Controlling Agent

In the present embodiment, the radiation-sensitive composition may contain an acid diffusion controlling agent having a function of controlling diffusion of an acid generated from an acid generating agent by radiation irradiation in a resist film to inhibit any unpreferable chemical reaction in an unexposed region or the like. By using such an acid diffusion controlling agent, the storage stability of a radiation-sensitive composition improves. Also, along with the improvement of the resolution, the line width change of a resist pattern due to variation in the post exposure delay time before radiation irradiation and the post exposure delay time after radiation irradiation can be inhibited, and the composition has extremely excellent process stability. Such an acid diffusion controlling agent includes a radiation degradable basic compound such as a nitrogen atom-containing basic compound, a basic sulfonium compound, and a basic iodonium compound. The acid diffusion controlling agent can be used alone or in combination of two or more kinds.

Examples of the above acid diffusion controlling agent include a nitrogen-containing organic compound, and a basic compound degradable by exposure. Examples of the above nitrogen-containing organic compound include a compound represented by the following general formula (10) :

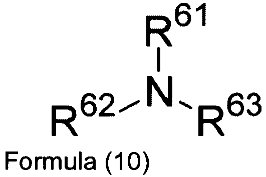

Examples of the above nitrogen-containing organic compound include the compound represented by the general formula (10) (hereinafter, referred to as a "nitrogen-containing compound (I)"), a diamino compound having two nitrogen atoms within the same molecule (hereinafter, referred to as a "nitrogen-containing compound (II)"), a polyamino compound or polymer having three or more nitrogen atoms (hereinafter, referred to as a "nitrogen-containing compound (III)"), an amide group-containing compound, a urea compound, and a nitrogen-containing heterocyclic compound. The acid diffusion controlling agent (E) may be used alone as one kind or may be used in combination of two or more kinds.

In the above general formula (10), R⁶¹, R⁶², and R⁶³ represent a hydrogen atom, a linear, branched or cyclic alkyl group, an aryl group, or an aralkyl group independently from each other. The above alkyl group, aryl group, or aralkyl group may be non-substituted or may be substituted with a hydroxyl group or the like. Herein, examples of the above linear, branched or cyclic alkyl group include the one of 1 to 15 carbon atoms, and preferably 1 to 10 carbon atoms. Specific examples thereof include a methyl group, an ethyl group, an n-propyl group, an isopropyl group, an n-butyl group, an isobutyl group, a sec-butyl group, a t-butyl group, an n-pentyl group, a neopentyl group, an n-hexyl group, a texyl group, an n-heptyl group, an n-octyl group, an n-ethylhexyl group, an n-nonyl group, and an n-decyl group. Examples of the above aryl group include the one of 6 to 12 carbon atoms. Specific examples thereof include a phenyl group, a tolyl group, a xylyl group, a cumenyl group, and a 1-naphthyl group. Furthermore, examples of the above aralkyl group include the one of 7 to 19 carbon atoms, and preferably 7 to 13 carbon atoms. Specific examples thereof include a benzyl group, an α-methylbenzyl group, a phenethyl group, and a naphthylmethyl group.

Specific examples of the above nitrogen-containing compound (I) include mono(cyclo)alkylamines such as n-hexylamine, n-heptylamine, n-octylamine, n-nonylamine, n-decylamine, n-dodecylamine, and cyclohexylamine; di(cyclo)alkylamines such as di-n-butylamine, di-n-pentylamine, di-n-hexylamine, di-n-heptylamine, di-n-octylamine, di-n-nonylamine, di-n-decylamine, methyl-n-dodecylamine, di-n-dodecylmethyl, cyclohexylmethylamine, and dicyclohexylamine; tri(cyclo)alkylamines such as triethylamine, tri-n-propylamine, tri-n-butylamine, tri-n-pentylamine, tri-n-hexylamine, tri-n-heptylamine, tri-n-octylamine, tri-n-nonylamine, tri-n-decylamine, dimethyl-n-dodecylamine, di-n-dodecylmethylamine, dicyclohexylmethylamine, and tricyclohexylamine; alkanolamines such as monoethanolamine, diethanolamine, and triethanolamine; and aromatic amines such as aniline, N-methylaniline, N,N-dimethylaniline, 2-methylaniline, 3-methylaniline, 4-methylaniline, 4-nitroaniline, diphenylamine, triphenylamine, and 1-naphthylamine.

Specific examples of the above nitrogen-containing compound (II) include ethylenediamine, N,N,N',N'-tetramethylethylenediamine, N,N,N',N'-tetrakis(2-hydroxypropyl)ethylenediamine, tetramethylenediamine, hexamethylenediamine, 4,4'-diaminodiphenylmethane, 4,4'-diaminodiphenyl ether, 4,4'-diaminobenzophenone, 4,4'-diaminodiphenylamine, 2,2-bis(4-aminophenyl)propane, 2-(3-aminophenyl)-2-(4-aminophenyl)propane, 2-(4-aminophenyl)-2-(3-hydroxyphenyl)propane, 2-(4-aminophenyl)-2-(4-hydroxyphenyl)propane, 1,4-bis[1-(4-aminophenyl)-1-methylethyl]benzene, and 1,3-bis[1-(4-aminophenyl)-1-methylethyl]benzene.

Specific examples of the above nitrogen-containing compound (III) include polymers of polyethyleneimine, polyarylamine, and N-(2-dimethylaminoethyl)acrylamide.

Specific examples of the above amide group-containing compound include formamide, N-methylformamide, N,N-dimethylformamide, acetamide, N-methylacetamide, N,N-dimethylacetamide, propioneamide, benzamide, pyrrolidone, and N-methylpyrrolidone.

Specific examples of the above urea compound include urea, methylurea, 1,1-dimethylurea, 1,3-dimethylurea, 1,1,3,3-tetramethylurea, 1,3-diphenylurea, and tri-n-butylthiourea.

Specific examples of the above nitrogen-containing heterocyclic compound include imidazoles such as imidazole, benzimidazole, 4-methylimidazole, 4-methyl-2-phenylimidazole, and 2-phenylbenzimidazole; pyridines such as pyridine, 2-methylpyridine, 4-methylpyridine, 2-ethylpyridine, 4-ethylpyridine, 2-phenylpyridine, 4-phenylpyridine, 2-methyl-4-phenylpyridine, nicotine, nicotinic acid, amide nicotinate, quinoline, 8-oxyquinoline, and acridine; and pyrazine, pyrazole, pyridazine, quinozaline, purine, pyrrolidine, piperidine, morpholine, 4-methylmorpholine, piperazine, 1,4-dimethylpiperazine, and 1,4-diazabicyclo[2.2.2]octane.

Examples of the radiation degradable basic compound can include a sulfonium compound represented by the following general formula (11-1), and an iodonium compound represented by the following general formula (11-2) :

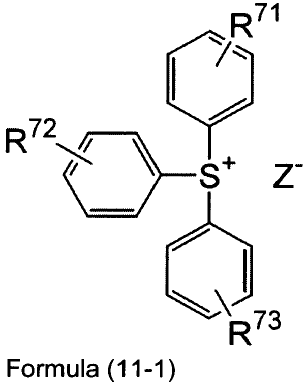

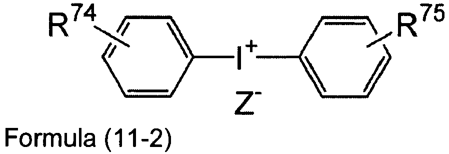

In the above general formulae (11-1) and (11-2), R⁷¹, R⁷², R⁷³, R⁷⁴, and R⁷⁵ represent a hydrogen atom, an alkyl group of 1 to 6 carbon atoms, an alkoxyl group of 1 to 6 carbon atoms, a hydroxyl group, or a halogen atom independently from each other. Z⁻ represents HO⁻, R-COO⁻ (R represents an alkyl group of 1 to 6 carbon atoms, an aryl group of 6 to 11 carbon atoms, or an alkaryl group of 7 to 12 carbon atoms), or an anion represented by the following general formula (11-3):

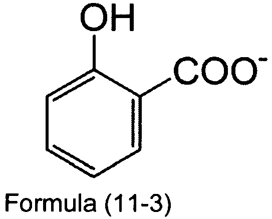

Specific examples of the above radiation degradable basic compound include triphenylsulfonium hydroxide, triphenylsulfonium acetate, triphenylsulfonium salicylate, diphenyl-4-hydroxyphenylsulfonium hydroxide, diphenyl-4-hydroxyphenylsulfonium acetate, diphenyl-4-hydroxyphenylsulfonium salicylate, bis(4-t-butylphenyl)iodonium hydroxide, bis(4-t-butylphenyl)iodonium acetate, bis(4-t-butylphenyl)iodonium hydroxide, bis(4-t-butylphenyl)iodonium acetate, bis(4-t-butylphenyl)iodonium salicylate, 4-t-butylphenyl-4-hydroxyphenyliodonium hydroxide, 4-t-butylphenyl-4-hydroxyphenyliodonium acetate, and 4-t-butylphenyl-4-hydroxyphenyliodonium salicylate.

The content of the above acid diffusion controlling agent is preferably 0 to 49% by mass of the total mass of the components except for the solvent (C), more preferably 0 to 10% by mass, still more preferably 0 to 5% by mass, and particularly preferably 0 to 3% by mass. When the content of the acid diffusion controlling agent falls within the above range, a decrease in resolution, and deterioration of the pattern shape and the dimension fidelity or the like tend to be able to be prevented. Moreover, even though the post exposure delay time from electron beam irradiation to heating after radiation irradiation becomes longer, the shape of the pattern upper layer portion is not deteriorated. When the content is 10% by mass or less, a decrease in sensitivity, and developability of the unexposed portion or the like tends to be able to be prevented. By using such an acid diffusion controlling agent, the storage stability of a radiation-sensitive composition improves. Also, along with improvement of the resolution, the line width change of a resist pattern due to variation in the post exposure delay time before radiation irradiation and the post exposure delay time after radiation irradiation can be inhibited, and the composition has extremely excellent process stability.

### (4) Dissolution Promoting Agent

A low molecular weight dissolution promoting agent is a component having a function of increasing the solubility of a resist base material represented by the formula (1) in a developing solution to moderately increase the dissolution rate of the resist base material upon developing, when the solubility of the resist base material is too low. The low molecular weight dissolution promoting agent can be used within the range of not deteriorating the effect of the present invention. Examples of the above dissolution promoting agent can include a low molecular weight phenolic compound, for example, bisphenols and tris(hydroxyphenyl)methane. These dissolution promoting agents can be used alone or in mixture of two or more kinds. The content of the dissolution promoting agent, which is arbitrarily adjusted according to the kind of the cyclic compound to be used, is preferably 0 to 49% by mass of the total mass of the components except for the solvent (C), more preferably 0 to 5% by mass, still more preferably 0 to 1% by mass, and particularly preferably 0% by mass.

### (5) Dissolution Controlling Agent

The dissolution controlling agent is a component having a function of controlling the solubility of the resist base material represented by the formula (1) in a developing solution to moderately decrease the dissolution rate upon developing, when the solubility of the resist base material is too high. As such a dissolution controlling agent, the one which does not chemically change in steps such as calcination of resist coating, radiation irradiation, and development is preferable.

Examples of the dissolution controlling agent include aromatic hydrocarbons such as phenanthrene, anthracene, and acenaphthene; ketones such as acetophenone, benzophenone, and phenyl naphtyl ketone; and sulfones such as methyl phenyl sulfone, diphenyl sulfone, and dinaphthyl sulfone. These dissolution controlling agents can be used alone or in two or more kinds.

The content of the dissolution controlling agent is arbitrarily adjusted according to the kind of the compound to be used, is preferably 0 to 49% by mass of the total mass of the components except for the solvent (C), more preferably 0 to 5% by mass, still more preferably 0 to 1% by mass, and particularly preferably 0% by mass.

### (6) Sensitizing Agent

The sensitizing agent is a component having a function of absorbing irradiated radiation energy, transmitting the energy to the acid generating agent, and thereby increasing the acid production amount, and improving the apparent sensitivity of a resist. Examples of such a sensitizing agent include benzophenones, biacetyls, pyrenes, phenothiazines, and fluorenes. These sensitizing agents can be used alone or in two or more kinds. The content of the sensitizing agent, which is arbitrarily adjusted according to the kind of the compound to be used, is preferably 0 to 49% by mass of the total mass of the components except for the solvent, more preferably 0 to 5% by mass, still more preferably 0 to 1% by mass, and particularly preferably 0% by mass.

### (7) Surfactant

The surfactant is a component having a function of improving coatability and striation of the radiation-sensitive composition of the present embodiment, and developability of a resist or the like. Such a surfactant may be any of anionic, cationic, nonionic or amphoteric. A preferable surfactant is a nonionic surfactant. The nonionic surfactant has a good affinity with a solvent used in production of radiation-sensitive compositions and more effects. Examples of the nonionic surfactant include, but not particularly limited to, a polyoxyethylene higher alkyl ethers, polyoxyethylene higher alkyl phenyl ethers, and higher fatty acid diesters of polyethylene glycol. Examples of commercially available products include, hereinafter by trade name, EFTOP (manufactured by Jemco Inc.), MEGAFAC (manufactured by DIC Corporation), Fluorad (manufactured by Sumitomo 3M Limited), AsahiGuard, Surflon (hereinbefore, manufactured by Asahi Glass Co., Ltd.), Pepole (manufactured by Toho Chemical Industry Co., Ltd.), KP (manufactured by Shin-Etsu Chemical Co., Ltd.), and Polyflow (manufactured by Kyoeisha Chemical Co., Ltd.). The content of the surfactant, which is arbitrarily adjusted according to the kind of the cyclic compound to be used, is preferably 0 to 49% by mass of the total mass of the components except for the solvent (C), more preferably 0 to 5% by mass, still more preferably 0 to 1% by mass, and particularly preferably 0% by mass.

### (8) Organic Carboxylic Acid or Oxo Acid of Phosphor or Derivative Thereof

For the purpose of prevention of sensitivity deterioration or improvement of a resist pattern shape and post exposure delay stability or the like, and as an additional optional component, an organic carboxylic acid or an oxo acid of phosphor or derivative thereof may be contained. The composition can be used in combination with the acid diffusion controlling agent, or may be used alone. As the organic carboxylic acid, for example, malonic acid, citric acid, malic acid, succinic acid, benzoic acid, and salicylic acid are preferable. Examples of the oxo acid of phosphor or derivative thereof include phosphoric acid or derivative thereof such as ester including phosphoric acid, di-n-butyl ester phosphate, and diphenyl ester phosphate; phosphonic acid or derivative thereof such as ester including phosphonic acid, dimethyl ester phosphonate, di-n-butyl ester phosphonate, phenylphosphonic acid, diphenyl ester phosphonate, and dibenzyl ester phosphonate; and phosphinic acid and derivative thereof such as ester including phosphinic acid and phenylphosphinic acid. Among these, phosphonic acid is particularly preferable.

The organic carboxylic acid or the oxo acid of phosphor or derivative thereof can be used alone or in combination of two or more kinds. The content of the organic carboxylic acid or the oxo acid of phosphor or derivative thereof, which is arbitrarily adjusted according to the kind of the cyclic compound to be used, is preferably 0 to 49% by mass of the total mass of the components except for the solvent (C), more preferably 0 to 5% by mass, still more preferably 0 to 1% by mass, and particularly preferably 0% by mass.

### (9) Other Additive Agent Excluding Above Dissolution Controlling Agent, Sensitizing Agent, Surfactant and Organic Carboxylic Acid or Oxo Acid of Phosphor, or Derivative Thereof

Furthermore, the radiation-sensitive composition of the present embodiment can contain one kind or two kinds or more of additive agents other than the above dissolution controlling agent, sensitizing agent, and surfactant, within the range of not inhibiting the purpose of the present invention, if required. Examples of such an additive agent include a dye, a pigment, and an adhesion aid. For example, the composition contains the dye or the pigment, and thereby a latent image of the exposed portion can be visualized and influence of halation upon exposure can be alleviated, which is preferable. The composition contains the adhesion aid, and thereby adhesiveness to a substrate can be improved, which is preferable. Furthermore, examples of other additive agents can include a halation preventing agent, a storage stabilizing agent, a defoaming agent, and a shape improving agent. Specific examples thereof can include 4-hydroxy-4'-methylchalkone.

The total amount of the above optional component (D) is 0 to 98% by mass of the total mass of the components except for the solvent (C), preferably 0 to 49% by mass, more preferably 0 to 10% by mass, still more preferably 0 to 5% by mass, and particularly preferably 0% by mass.

The content of the radiation-sensitive composition of the present embodiment (the resist base material (A)/the optically active diazonaphthoquinone compound (B)/the optional component (D) is 1 to 99/99 to 1/0 to 98 in % by mass based on all components except for the solvent, preferably 5 to 95/95 to 5/0 to 49, more preferably 10 to 90/90 to 10/0 to 10, still more preferably 20 to 80/80 to 20/0 to 5, and particularly preferably 25 to 75/75 to 25/0. The content ratio of each component is selected from each range so that the summation thereof is 100% by mass. By the above content ratio, roughness as well as performance such as sensitivity and resolution is excellent.

The radiation-sensitive composition of the present embodiment can contain a resin as the optional component (D) within the range of not inhibiting the purpose of the present invention. The resin includes a novolac resin, polyvinyl phenols, polyacrylic acid, polyvinyl alcohol, a styrene-maleic anhydride resin, and a polymer containing an acrylic acid, vinyl alcohol, or vinylphenol as a monomeric unit, or derivative thereof. The content of the resin, which is arbitrarily adjusted according to the kind of the compound represented by the formula (1) to be used, is preferably 30 parts by mass or less per 100 parts by mass of the compound, more preferably 10 parts by mass or less, still more preferably 5 parts by mass or less, and particularly preferably 0 part by mass.

### [Resist Pattern Formation Method]

The present invention relates to a resist pattern formation method including steps of forming a resist film on a substrate using the above radiation-sensitive composition of the present embodiment, exposing the resist film, and developing the resist film, thereby forming a resist pattern. The resist pattern of the present embodiment can also be formed as an upper layer resist in a multilayer process.

In order to form a resist pattern, a resist film is formed by coating a conventionally publicly known substrate with the above radiation-sensitive composition of the present embodiment using a coating means such as spin coating, flow casting coating, and roll coating (step of forming a resist film on a substrate). For example, the radiation-sensitive composition of the present embodiment can form an amorphous film by spin coating. The conventionally publically known substrate is not particularly limited. For example, a substrate for electronic components, and the one having a predetermined wiring pattern formed thereon, or the like can be exemplified. More specific examples include a substrate made of a metal such as a silicon wafer, copper, chromium, iron and aluminum, and a glass substrate. Examples of a wiring pattern material include copper, aluminum, nickel, and gold. Also if required, the substrate may be a substrate having an inorganic and/or organic film provided thereon. Examples of the inorganic film include an inorganic antireflection film (inorganic BARC). Examples of the organic film include an organic antireflection film (organic BARC). Surface treatment with hexamethylene disilazane or the like may be conducted.

Next, the coated substrate is heated if required. The heating conditions vary according to the content composition of the radiation-sensitive composition, or the like, but are preferably 20 to 250°C, and more preferably 20 to 150°C. By heating, the adhesiveness of a resist to a substrate may improve, which is preferable. Then, the resist film is exposed to a desired pattern by any radiation selected from the group consisting of visible light, ultraviolet, excimer laser, electron beam, extreme ultraviolet (EUV), X-ray, and ion beam (step of exposing the resist film). The exposure conditions or the like are arbitrarily selected according to the compounding composition of the radiation-sensitive composition, or the like. In the present invention, in order to stably form a fine pattern with a high degree of accuracy in exposure, the resist film is preferably heated after radiation irradiation. The heating conditions vary according to the compounding composition of the radiation-sensitive composition, or the like, but are preferably 20 to 250°C, and more preferably 20 to 150°C.

Next, by developing the exposed resist film in a developing solution, a predetermined resist pattern is formed (step of developing the resist film, thereby forming a resist pattern). As the developing solution, a solvent having a solubility parameter (SP value) close to that of the resist base material of the formula (1) to be used is preferably selected. A polar solvent such as a ketone-based solvent, an ester-based solvent, an alcohol-based solvent, an amide-based solvent, and an ether-based solvent; and a hydrocarbon-based solvent, or an alkaline aqueous solution can be used.

Examples of the ketone-based solvent include 1-octanone, 2-octanone, 1-nonanone, 2-nonanone, acetone, 4-heptanone, 1-hexanone, 2-hexanone, diisobutyl ketone, cyclohexanone, methylcyclohexanone, phenylacetone, methyl ethyl ketone, methyl isobutyl ketone, acetylacetone, acetonylacetone, ionone, diacetonyl alcohol, acetyl carbinol, acetophenone, methyl naphthyl ketone, isophorone, and propylene carbonate.

Examples of the ester-based solvent include methyl acetate, butyl acetate, ethyl acetate, isopropyl acetate, amyl acetate, propylene glycol monomethyl ether acetate, ethylene glycol monoethyl ether acetate, diethylene glycol monobutyl ether acetate, diethylene glycol monoethyl ether acetate, ethyl-3-ethoxypropionate, 3-methoxybutyl acetate, 3-methyl-3-methoxybutyl acetate, methyl formate, ethyl formate, butyl formate, propyl formate, ethyl lactate, butyl lactate, and propyl lactate.

Examples of the alcohol-based solvent include an alcohol such as methyl alcohol, ethyl alcohol, n-propyl alcohol, isopropyl alcohol (2-propanol), n-butyl alcohol, sec-butyl alcohol, tert-butyl alcohol, isobutyl alcohol, n-hexyl alcohol, 4-methyl-2-pentanol, n-heptyl alcohol, n-octyl alcohol, and n-decanol; a glycol-based solvent such as ethylene glycol, diethylene glycol, and triethylene glycol; and a glycol ether-based solvent such as ethylene glycol monomethyl ether, propylene glycol monomethyl ether, ethylene glycol monoethyl ether, propylene glycol monoethyl ether, diethylene glycol monomethyl ether, triethylene glycol monoethyl ether, and methoxymethyl butanol.

Examples of the ether-based solvent include dioxane and tetrahydrofuran in addition to the above glycol ether-based solvents.

Examples of the amide-based solvent which can be used include N-methyl-2-pyrrolidone, N,N-dimethylacetamide, N,N-dimethylformamide, phosphoric hexamethyltriamide, and 1,3-dimethyl-2-imidazolidinone.

Examples of the hydrocarbon-based solvent include an aromatic hydrocarbon-based solvent such as toluene and xylene; and an aliphatic hydrocarbon-based solvent such as pentane, hexane, octane, and decane.

A plurality of above solvents may be mixed, or the solvent may be used by mixing the solvent with a solvent other than those described above or water within the range having performance. However, in order to exhibit more than enough effect of the present invention, the water content ratio as the whole developing solution is less than 70% by mass, preferably less than 50% by mass, more preferably less than 30% by mass, and still more preferably less than 10% by mass. Particularly preferably, the developing solution is substantially moisture free. That is, the content of the organic solvent in the developing solution is 30% by mass or more and 100% by mass or less based on the total amount of the developing solution, preferably 50% by mass or more and 100% by mass or less, more preferably 70% by mass or more and 100% by mass or less, still more preferably 90% by mass or more and 100% by mass or less, and particularly preferably 95% by mass or more and 100% by mass or less.

Examples of the alkaline aqueous solution include an alkaline compound such as mono-, di- or tri-alkylamines, mono-, di- or tri-alkanolamines, heterocyclic amines, tetramethyl ammonium hydroxide (TMAH), and choline.

Particularly, the developing solution containing at least one kind of solvent selected from a ketone-based solvent, an ester-based solvent, an alcohol-based solvent, an amide-based solvent, and an ether-based solvent improves resist performance such as resolution and roughness of the resist pattern, which is preferable.

The vapor pressure of the developing solution is preferably 5 kPa or less at 20°C, more preferably 3 kPa or less, and particularly preferably 2 kPa or less. The evaporation of the developing solution on the substrate or in a developing cup is inhibited by setting the vapor pressure of the developing solution to 5 kPa or less, to improve temperature uniformity within a wafer surface, thereby resulting in improvement in size uniformity within the wafer surface.

Specific examples of developing solution having a vapor pressure of 5 kPa or less include a ketone-based solvent such as 1-octanone, 2-octanone, 1-nonanone, 2-nonanone, 4-heptanone, 2-hexanone, diisobutyl ketone, cyclohexanone, methylcyclohexanone, phenylacetone, and methyl isobutyl ketone; an ester-based solvent such as butyl acetate, amyl acetate, propylene glycol monomethyl ether acetate, ethylene glycol monoethyl ether acetate, diethylene glycol monobutyl ether acetate, diethylene glycol monoethyl ether acetate, ethyl-3-ethoxy propionate, 3-methoxy butyl acetate, 3-methyl-3-methoxy butyl acetate, butyl formate, propyl formate, ethyl lactate, butyl lactate, and propyl lactate; an alcohol-based solvent such as n-propyl alcohol, isopropyl alcohol, n-butyl alcohol, sec-butyl alcohol, tert-butyl alcohol, isobutyl alcohol, n-hexyl alcohol, 4-methyl-2-pentanol, n-heptyl alcohol, n-octyl alcohol, and n-decanol; a glycol-based solvent such as ethylene glycol, diethylene glycol, and triethylene glycol; a glycol ether-based solvent such as ethylene glycol monomethyl ether, propylene glycol monomethyl ether, ethylene glycol monoethyl ether, propylene glycol monoethyl ether, diethylene glycol monomethyl ether, triethylene glycol monoethyl ether, and methoxymethyl butanol; an ether-based solvent such as tetrahydrofuran; an amide-based solvent such as N-methyl-2-pyrrolidone, N,N-dimethylacetamide, and N,N-dimethylformamide; an aromatic hydrocarbon-based solvent such as toluene and xylene; and an aliphatic hydrocarbon-based solvent such as octane and decane.

Specific examples of developing solution having a vapor pressure of 2 kPa or less which is a particularly preferable range include a ketone-based solvent such as 1-octanone, 2-octanone, 1-nonanone, 2-nonanone, 4-heptanone, 2-hexanone, diisobutyl ketone, cyclohexanone, methylcyclohexanone, and phenylacetone; an ester-based solvent such as butyl acetate, amyl acetate, propylene glycol monomethyl ether acetate, ethylene glycol monoethyl ether acetate, diethylene glycol monobutyl ether acetate, diethylene glycol monoethyl ether acetate, ethyl-3-ethoxy propionate, 3-methoxy butyl acetate, 3-methyl-3-methoxy butyl acetate, ethyl lactate, butyl lactate, and propyl lactate; an alcohol-based solvent such as n-butyl alcohol, sec-butyl alcohol, tert-butyl alcohol, isobutyl alcohol, n-hexyl alcohol, 4-methyl-2-pentanol, n-heptyl alcohol, n-octyl alcohol, and n-decanol; a glycol-based solvent such as ethylene glycol, diethylene glycol, and triethylene glycol; a glycol ether-based solvent such as ethylene glycol monomethyl ether, propylene glycol monomethyl ether, ethylene glycol monoethyl ether, propylene glycol monoethyl ether, diethylene glycol monomethyl ether, triethylene glycol monoethyl ether, and methoxymethyl butanol; an amide-based solvent such as N-methyl-2-pyrrolidone, N,N-dimethylacetamide, and N,N-dimethylformamide; an aromatic hydrocarbon-based solvent such as xylene; and an aliphatic hydrocarbon-based solvent such as octane and decane.

To the developing solution, a surfactant can be added in an appropriate amount, if required. The surfactant is not particularly limited but, for example, an ionic or nonionic fluorine-based and/or silicon-based surfactant can be used. Examples of the fluorine-based and/or silicon-based surfactant include the surfactants described in Japanese Patent Application Laid-Open Nos. 62-36663, 61-226746, 61-226745, 62-170950, 63-34540, 7-230165, 8-62834, 9-54432, and 9-5988, and U.S. Pat. Nos. 5,405,720, 5,360,692, 5,529,881, 5,296,330, 5,436,098, 5,576,143, 5,294,511, and 5,824,451. The surfactant is preferably a nonionic surfactant. The nonionic surfactant is not particularly limited, but a fluorine-based surfactant or a silicon-based surfactant is more preferably used.

The amount of the surfactant used is usually 0.001 to 5% by mass based on the total amount of the developing solution, preferably 0.005 to 2% by mass, and more preferably 0.01 to 0.5% by mass.

The development method is, for example, a method for dipping a substrate in a bath filled with a developing solution for a fixed time (dipping method), a method for raising a developing solution on a substrate surface by the effect of a surface tension and keeping it still for a fixed time, thereby conducting the development (puddle method), a method for spraying a developing solution on a substrate surface (spraying method), and a method for continuously ejecting a developing solution on a substrate rotating at a constant speed while scanning a developing solution ejecting nozzle at a constant rate (dynamic dispense method), or the like may be applied. The time for conducting the pattern development is not particularly limited, but is preferably 10 seconds to 90 seconds.

After the step of conducting development, a step of stopping the development by the replacement with another solvent may be practiced.

A step of rinsing the resist film with a rinsing solution containing an organic solvent is preferably provided after the development.

The rinsing solution used in the rinsing step after development is not particularly limited as long as the rinsing solution does not dissolve the formed resist pattern. A solution containing a general organic solvent or water may be used as the rinsing solution. As the rinsing solution, a rinsing solution containing at least one kind of organic solvent selected from a hydrocarbon-based solvent, a ketone-based solvent, an ester-based solvent, an alcohol-based solvent, an amide-based solvent, and an ether-based solvent is preferably used. More preferably, after development, a step of rinsing the film by using a rinsing solution containing at least one kind of organic solvent selected from the group consisting of a ketone-based solvent, an ester-based solvent, an alcohol-based solvent and an amide-based solvent is conducted. Still more preferably, after development, a step of rinsing the film by using a rinsing solution containing an alcohol-based solvent or an ester-based solvent is conducted. Further more preferably, after development, a step of rinsing the film by using a rinsing solution containing a monohydric alcohol is conducted. Particularly preferably, after development, a step of rinsing the film by using a rinsing solution containing a monohydric alcohol having 5 or more carbon atoms is conducted. The time for rinsing the pattern is not particularly limited, but is preferably 10 seconds to 90 seconds.

Herein, examples of the monohydric alcohol used in the rinsing step after development include a linear, branched or cyclic monohydric alcohol. Specifically, 1-butanol, 2-butanol, 3-methyl-1-butanol, tert-butyl alcohol, 1-pentanol, 2-pentanol, 1-hexanol, 4-methyl-2-pentanol, 1-heptanol, 1-octanol, 2-hexanol, cyclopentanol, 2-heptanol, 2-octanol, 3-hexanol, 3-heptanol, 3-octanol, and 4-octanol or the like can be used. As a particularly preferable monohydric alcohol having 5 or more carbon atoms, 1-hexanol, 2-hexanol, 4-methyl-2-pentanol, 1-pentanol, and 3-methyl-1-butanol or the like can be used.

A plurality of these components may be mixed, or the component may be used by mixing the component with an organic solvent other than those described above.

The water content ratio in the rinsing solution is preferably 10% by mass or less, more preferably 5% by mass or less, and particularly preferably 3% by mass or less. By setting the water content ratio to 10% by mass or less, better development characteristics can be obtained.

The vapor pressure at 20°C of the rinsing solution used after development is preferably 0.05 kPa or more and 5 kPa or less, more preferably 0.1 kPa or more and 5 kPa or less, and most preferably 0.12 kPa or more and 3 kPa or less. By setting the vapor pressure of the rinsing solution to 0.05 kPa or more and 5 kPa or less, the temperature uniformity in the wafer surface is further enhanced and moreover, swelling due to permeation of the rinsing solution is further inhibited. As a result, the dimensional uniformity in the wafer surface is further improved.

The rinsing solution may also be used after adding an appropriate amount of a surfactant to the rinsing solution.

In the rinsing step, the wafer after development is rinsed using the organic solvent-containing rinsing solution. The method for rinsing treatment is not particularly limited. However, for example, a method for continuously ejecting a rinsing solution on a substrate spinning at a constant speed (spin coating method), a method for dipping a substrate in a bath filled with a rinsing solution for a fixed time (dipping method), and a method for spraying a rinsing solution on a substrate surface (spraying method), or the like can be applied. Above all, it is preferable to conduct the rinsing treatment by the spin coating method and after the rinsing, spin the substrate at a rotational speed of 2,000 rpm to 4,000 rpm, to remove the rinsing solution from the substrate surface.

After forming the resist pattern, a pattern wiring substrate is obtained by etching. Etching can be conducted by a publicly known method such as dry etching using plasma gas, and wet etching with an alkaline solution, a cupric chloride solution, and a ferric chloride solution or the like.

After forming the resist pattern, plating can also be conducted. Examples of the above plating method include copper plating, solder plating, nickel plating, and gold plating.

The remaining resist pattern after etching can be peeled by an organic solvent. Examples of the above organic solvent include PGMEA (propylene glycol monomethyl ether acetate), PGME (propylene glycol monomethyl ether), and EL (ethyl lactate). Examples of the above peeling method include a dipping method and a spraying method. A wiring substrate having a resist pattern formed thereon may be a multilayer wiring substrate, and may have a small diameter through hole.

The wiring substrate obtained in the present embodiment can also be formed by a method for forming a resist pattern, then depositing a metal in vacuum, and subsequently dissolving the resist pattern in a solution, i.e., a liftoff method.

### Examples

The embodiment of the present invention will be more specifically described with reference to examples below. However, the present invention is not limited to these examples. Hereinafter, the structure of a compound in production examples was confirmed by ¹H-NMR measurement.

### (Carbon Concentration and Oxygen Concentration)

A carbon concentration and an oxygen concentration (% by mass) were measured by organic elemental analysis.

Apparatus: CHN Corder MT-6 (manufactured by Yanaco Analytical Instruments Inc.)

### (Molecular Weight)

A molecular weight was measured using "Acquity UPLC/MALDI-Synapt HDMS" manufactured by Water Corporation according to LC-MS analysis.

### (Thermal Reduction Temperature)

"EXSTAR 6000 DSC apparatus" manufactured by SII NanoTechnology Inc. was used. About 5 mg of a sample was placed in an unsealed container made of aluminum, and the temperature was raised to 500°C at a temperature increase rate of 10°C/min in a nitrogen gas (30 mL/min) stream.
In this operation, 10% thermal reduction temperature was measured.

### (Solubility)

The dissolution amount of the compound in 1-methoxy-2-propanol (PGME) and propylene glycol monomethyl ether acetate (PGMEA) was measured at 23°C, and the results were evaluated according to the following criteria.
Evaluation A: 20% by mass or more
Evaluation B: 10% by mass or more and less than 20% by mass
Evaluation C: less than 10% by mass

### (Production Example 1) Synthesis of BiF-I-1

A container (internal capacity: 200 mL) equipped with a stirrer, a condenser tube, and a burette was prepared. In this container, 30 g (161 mmol) of 4,4'-biphenol (reagent manufactured by Tokyo Kasei Kogyo Co., Ltd.), 15 g (65 mmol) of 4-iodobenzaldehyde (reagent manufactured by Tokyo Kasei Kogyo Co., Ltd.), and 100 mL of 4-butyrolactone were charged, and 3.9 g (21 mmol) of p-toluenesulfonic acid (reagent manufactured by Kanto Chemical Co., Inc.) was added thereto to prepare a reaction solution. This reaction solution was stirred at 90°C for 3 hours to perform reaction. Next, the reaction solution was concentrated. The reaction product was precipitated by the addition of 50 g of heptane. After cooling to room temperature, the precipitates were separated by filtration. The solid matter obtained by filtration was dried, and then separated and purified by column chromatography to obtain 4.2 g of the objective compound (BiF-I-1) represented by the following formula.

The following peaks were found by 400 MHz-¹H-NMR, and the compound was confirmed to have a chemical structure of the following formula.
¹H-NMR: (d-DMSO, internal standard TMS)
δ (ppm) 9.4 (4H, O-H), 6.8 to 7.8 (22H, Ph-H), 6.2 (1H, C-H)

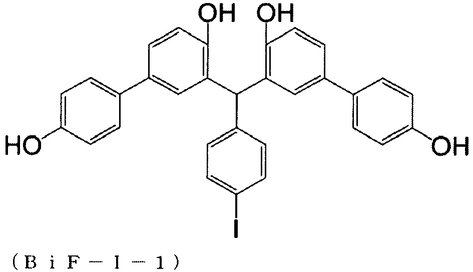

As a result of organic elemental analysis, the obtained compound (BiF-I-1) had a carbon concentration of 82.9% and an oxygen concentration of 11.8%.

As a result of measuring the molecular weight of the obtained compound by the above method, it was 516.

As a result of thermogravimetry (TG), the 10% thermal reduction temperature of the obtained compound (BiF-I-1) was 400°C or higher. Therefore, this compound was evaluated as having high heat resistance and being applicable to baking at a high temperature.

As a result of evaluating solubility in PGME and PGMEA, it was 30 % by mass or more (evaluation A), and the compound (BiF-I-1) was evaluated as having excellent solubility. Therefore, the compound (BiF-I-1) was evaluated as having high storage stability in a solution state and being sufficiently applicable to even an edge bead removal solution (PGME/PGMEA mixed solution) widely used in the finer processing of semiconductors.

### (Production Example 2) Synthesis of BiF-I-2

A container (internal capacity: 200 mL) equipped with a stirrer, a condenser tube, and a burette was prepared. In this container, 30 g (161 mmol) of 4,4-biphenol (reagent manufactured by Tokyo Kasei Kogyo Co., Ltd.), 15 g (54 mmol) of 5-iodovanillin (reagent manufactured by Tokyo Kasei Kogyo Co., Ltd.), and 100 mL of 4-butyrolactone were charged, and 3.9 g (21 mmol) of p-toluenesulfonic acid (reagent manufactured by Kanto Chemical Co., Inc.) was added thereto to prepare a reaction solution. This reaction solution was stirred at 90°C for 3 hours to perform reaction. Next, the reaction solution was concentrated. The reaction product was precipitated by the addition of 50 g of heptane. After cooling to room temperature, the precipitates were separated by filtration. The solid matter obtained by filtration was dried, and then separated and purified by column chromatography to obtain 5.1 g of the objective compound (BiF-I-2) represented by the following formula.

The following peaks were found by 400 MHz-¹H-NMR, and the compound was confirmed to have a chemical structure of the following formula.
¹H-NMR: (d-DMSO, internal standard TMS)
δ (ppm) 9.4 (5H, O-H), 6.8 to 7.8 (16H, Ph-H), 6.2 (1H, C-H), 3.7 (3H, O-CH₃)

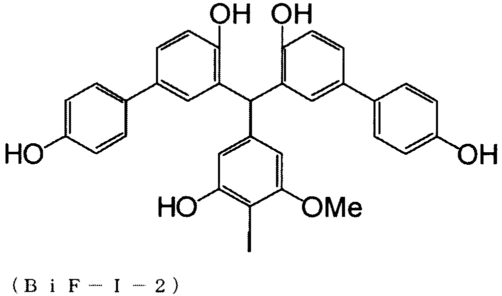

As a result of organic elemental analysis, the obtained compound (BiF-I-2) had a carbon concentration of 82.9% and an oxygen concentration of 11.8%.

As a result of measuring the molecular weight of the obtained compound by the above method, it was 516.

As a result of thermogravimetry (TG), the 10% thermal reduction temperature of the obtained compound (BiF-I-2) was 400°C or higher. Therefore, this compound was evaluated as having high heat resistance and being applicable to baking at a high temperature.

As a result of evaluating solubility in PGME and PGMEA, it was 30 % by mass or more (evaluation A), and the compound (BiF-I-2) was evaluated as having excellent solubility. Therefore, the compound (BiF-I-2) was evaluated as having high storage stability in a solution state and being sufficiently applicable to even an edge bead removal solution (PGME/PGMEA mixed solution) widely used in the finer processing of semiconductors.

### (Production Example 3) Synthesis of BiF-I-3

A container (internal capacity: 200 mL) equipped with a stirrer, a condenser tube, and a burette was prepared. In this container, 30 g (161 mmol) of 4,4'-biphenol (reagent manufactured by Tokyo Kasei Kogyo Co., Ltd.), 15 g (65 mmol) of 3-iodobenzaldehyde (reagent manufactured by Tokyo Kasei Kogyo Co., Ltd.), and 100 mL of 4-butyrolactone were charged, and 3.9 g (21 mmol) of p-toluenesulfonic acid (reagent manufactured by Kanto Chemical Co., Inc.) was added thereto to prepare a reaction solution. This reaction solution was stirred at 90°C for 3 hours to perform reaction. Next, the reaction solution was concentrated. The reaction product was precipitated by the addition of 50 g of heptane. After cooling to room temperature, the precipitates were separated by filtration. The solid matter obtained by filtration was dried, and then separated and purified by column chromatography to obtain 4.2 g of the objective compound (BiF-I-3) represented by the following formula.

As a result of measuring the molecular weight of the obtained compound by the above method, it was 586.

The following peaks were found by 400 MHz-1H-NMR, and the compound was confirmed to have a chemical structure of the following formula.
¹H-NMR: (d-DMSO, internal standard TMS)
δ (ppm) 9.4 (4H, O-H), 6.5 to 7.8 (18H, Ph-H), 6.4 (1H, C-H)

As a result of thermogravimetry (TG), the 10% thermal reduction temperature of the obtained compound (BiF-I-3) was 300°C or higher. Therefore, this compound was evaluated as having high heat resistance and being applicable to baking at a high temperature.

As a result of evaluating solubility in PGME and PGMEA, it was 30 % by mass or more (evaluation A), and the compound (BiF-I-3) was evaluated as having excellent solubility. Therefore, the compound (BiF-I-3) was evaluated as having high storage stability in a solution state and being sufficiently applicable to even an edge bead removal solution (PGME/PGMEA mixed solution) widely used in the finer processing of semiconductors.

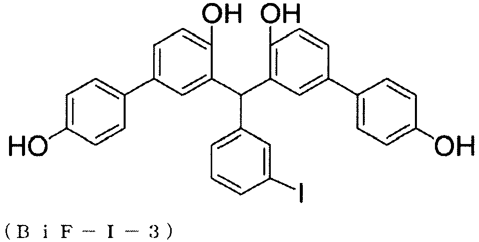

### (Production Example 4) Synthesis of Resin (BiFR-I-1)

A four necked flask (internal capacity: 1 L) equipped with a Dimroth condenser tube, a thermometer, and a stirring blade and having a detachable bottom was prepared. In this four necked flask, 41.0 g (70 mmol) of BiF-I-1 obtained in Production Example 1 (manufactured by Mitsubishi Gas Chemical Company, Inc.), 21.0 g (280 mmol as formaldehyde) of 40% by mass of an aqueous formalin solution (manufactured by Mitsubishi Gas Chemical Company, Inc.), and 0.97 mL of 98% by mass of sulfuric acid (manufactured by Kanto Chemical Co., Inc.) were charged in a nitrogen stream, and reacted for 7 hours while refluxed at 100°C at normal pressure. Subsequently, 180.0 g of o-xylene (special grade reagent manufactured by Wako Pure Chemical Industries, Ltd.) was added as a diluting solvent to the reaction solution, and it was left at rest, followed by removal of an aqueous phase as a lower phase. Neutralization and washing with water were further performed, and o-xylene was distilled off under reduced pressure to obtain 52.2 g of a brown solid resin (BiFR-I-1).

The obtained resin (BiFR-I-1) had Mn of 1685, Mw of 3120, and Mw/Mn of 1.85.

As a result of thermogravimetry (TG), the 10% thermal reduction temperature of the obtained resin (BiFR-I-1) was 300°C or higher. Therefore, this resin was evaluated as being applicable to baking at a high temperature.

As a result of evaluating solubility in PGME and PGMEA, it was 10% by mass or more (evaluation A), and the resin (BiFR-I-1) was evaluated as having excellent solubility.

### (Production Example 5) Synthesis of Resin (BiFR-I-2)

A four necked flask (internal capacity: 1 L) equipped with a Dimroth condenser tube, a thermometer, and a stirring blade and having a detachable bottom was prepared. In this four necked flask, 41.0 g (70 mmol) of BiF-I-1 obtained in Production Example 1 (manufactured by Mitsubishi Gas Chemical Company, Inc.), 50.9 g (280 mmol) of 4-biphenylaldehyde (manufactured by Mitsubishi Gas Chemical Company, Inc.), 100 mL of anisole (manufactured by Kanto Chemical Co., Inc.), and 10 mL of oxalic acid dihydrate (manufactured by Kanto Chemical Co., Inc.) were charged in a nitrogen stream, and reacted for 7 hours while refluxed at 100°C at normal pressure. Subsequently, 180.0 g of o-xylene (special grade reagent manufactured by Wako Pure Chemical Industries, Ltd.) was added as a diluting solvent to the reaction solution, and it was left at rest, followed by removal of an aqueous phase as a lower phase. Neutralization and washing with water were further performed, and the solvent of the organic phase and unreacted 4-biphenylaldehyde were distilled off under reduced pressure to obtain 68.2 g of a brown solid resin (BiFR-I-2).

The obtained resin (BiFR-I-2) had Mn of 2080, Mw of 3650, and Mw/Mn of 1.75.

As a result of thermogravimetry (TG), the 10% thermal reduction temperature of the obtained resin (BiFR-I-2) was 300°C or higher. Therefore, this resin was evaluated as being applicable to baking at a high temperature.

As a result of evaluating solubility in PGME and PGMEA, it was 10% by mass or more (evaluation A), and the resin (BiFR-I-2) was evaluated as having excellent solubility.

### [Examples and Comparative Example]

### (1) Evaluation of Resist Performance

The components described in Table 1 below were prepared into a homogeneous solution, which was then filtered through a Teflon(R) membrane filter with a pore diameter of 0.1 µm to prepare a radiation-sensitive composition. Each composition was evaluated.

**[Table 1]**

| | Resist base material (A) | Optically active compound (B) | Solvent |
|---|---|---|---|
| | (g) | (g) | (g) |
| Example 1 | BiF-I-1 | P-1 | S-1 |
| | 0.5 | 1.5 | 30.0 |
| Example 2 | BiF-I-2 | P-1 | S-1 |
| | 0.5 | 1.5 | 30.0 |
| Example 3 | BiF-I-3 | P-1 | S-1 |
| | 0.5 | 1.5 | 30.0 |
| Example 4 | BiFR-I-1 | P-1 | S-1 |
| | 0.5 | 1.5 | 30.0 |
| Example 5 | BiFR-I-2 | P-1 | S-1 |
| | 0.5 | 1.5 | 30.0 |
| Example 6 | BiF-I-1 | P-2 | S-1 |
| | 0.5 | 1.5 | 30.0 |
| Example 7 | BiF-I-1 | P-3 | S-1 |
| | 0.5 | 1.5 | 30.0 |
| Example 8 | BiF-I-1 | P-4 | S-1 |
| | 0.5 | 1.5 | 30.0 |
| Comparative Example 1 | PHS-1 | P-1 | S-1 |
| | 0.5 | 1.5 | 30.0 |

In Table 1, the following was used as the resist base material of Comparative Example 1.
   PHS-1: polyhydroxystyrene (Mw = 8000) (Sigma-Aldrich Co., LLC)
In Table 1, the following was used as the optically active compound (B).
   P-1: naphthoquinonediazide based photosensitizing agent of the following chemical structural formula (G) (4NT-300, Toyo Gosei Co., Ltd.)
   P-2: naphthoquinonediazide based photosensitizing agent of the following chemical structural formula (G-1) (TS-200, Sanbo Chemical Ind., Ltd.)
   P-3: naphthoquinonediazide based photosensitizing agent of the following chemical structural formula (G-2) (TKE1-510, Sanbo Chemical Ind., Ltd.)
   P-4: naphthoquinonediazide based photosensitizing agent of the following chemical structural formula (G-3) (PQ-614, Sanbo Chemical Ind., Ltd.)

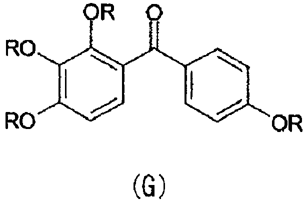

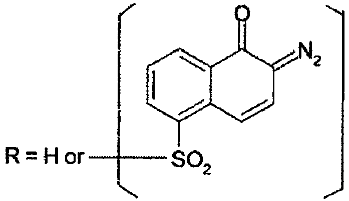

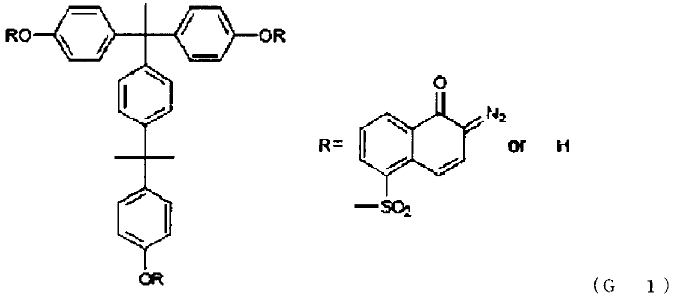

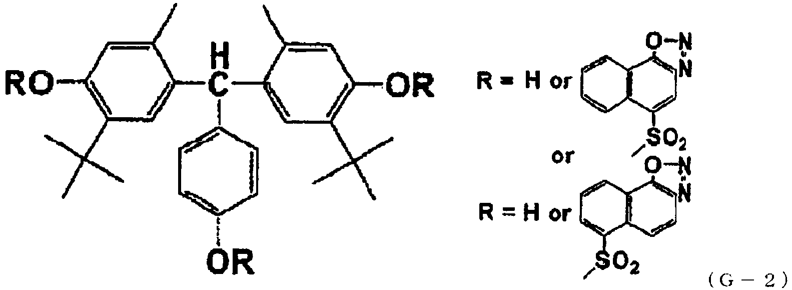

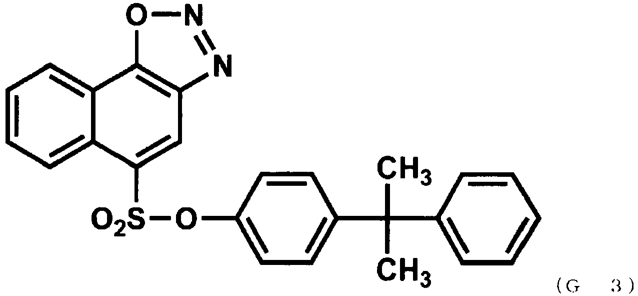

The following was used as the solvent.
S-1: propylene glycol monomethyl ether (Tokyo Kasei Kogyo Co., Ltd.)

A clean silicon wafer was spin coated with a radiation-sensitive composition, and then prebaked (PB) before exposure in an oven of 110°C to form a resist film with a thickness of 200 nm. The resist film was exposed to ultraviolet using an ultraviolet exposure apparatus (mask aligner MA-10 manufactured by Mikasa Co., Ltd.). The ultraviolet lamp used was a super high pressure mercury lamp (relative intensity ratio: g-ray:h-ray:i-ray:j-ray = 100:80:90:60). After irradiation, it was heated at 110°C for 90 seconds, and immersed in 2.38% by mass tetramethylammonium hydroxide (TMAH) alkaline developing solution for 60 seconds for development. Subsequently, it was washed with ultrapure water for 30 seconds, and dried to form a 5 µm positive type resist pattern.

The obtained line and space were observed by a scanning electron microscope (S-4800 manufactured by Hitachi High-Technologies Corporation). As for the line edge roughness, a pattern having asperities of less than 50 nm was evaluated as goodness.

The resist film before ultraviolet irradiation and the resist film after ultraviolet irradiation obtained by the above method were immersed in 2.38% by mass tetramethylammonium hydroxide (TMAH) alkaline developing solution for 60 seconds for development. Subsequently, they were washed with ultrapure water for 30 seconds, and dried, and their film thicknesses were measured using an automatic ellipsometer (MARY-102 manufactured by Five Lab Co., Ltd.). Dissolution rates of 5 angstrom/sec or less before ultraviolet irradiation and 10 angstrom/sec or more after ultraviolet irradiation were evaluated as goodness.

In the resists of Examples 1 to 8, resist patterns with good resolution of 5 µm could be obtained. The roughness of the patterns was also small and good.

The dissolution rates of the resist films in the developing solution at 23°C were 5 angstrom/sec or less before ultraviolet irradiation and 10 angstrom/sec or more after ultraviolet irradiation, and were good.

In the resist of Comparative Example 1, a resist pattern with good resolution of 5 µm could be obtained. However, the roughness of the pattern was large and poor.

As described above, the radiation-sensitive composition in the present example can form a resist pattern having smaller roughness and a better shape, as compared with the composition of comparative example. As long as the above configuration of the present embodiment is met, compositions other than those described in examples also exhibit the same effects.

The disclosure of Japanese Patent Application No. 2015-069988 filed with JPO on March 30, 2015 is hereby incorporated by reference in its entirety

All publications, patent applications, and technical standards described herein are hereby incorporated by reference to the same extent as if each literature, patent application, and technical standard are specifically and individually indicated to be hereby incorporated by reference.

### Industrial Applicability

The present invention can provide a specific radiation-sensitive composition useful as a resist materials. The composition is preferably used in resist pattern formation.

## Claims

1. A radiation-sensitive composition comprising a resist base material (A), an optically active diazonaphthoquinone compound (B), and a solvent (C), wherein the content of the solvent (C) in the composition is 20 to 99% by mass, the content of components except for the solvent (C) is 1 to 80% by mass, and the resist base material (A) is a compound represented by the following formula (1): 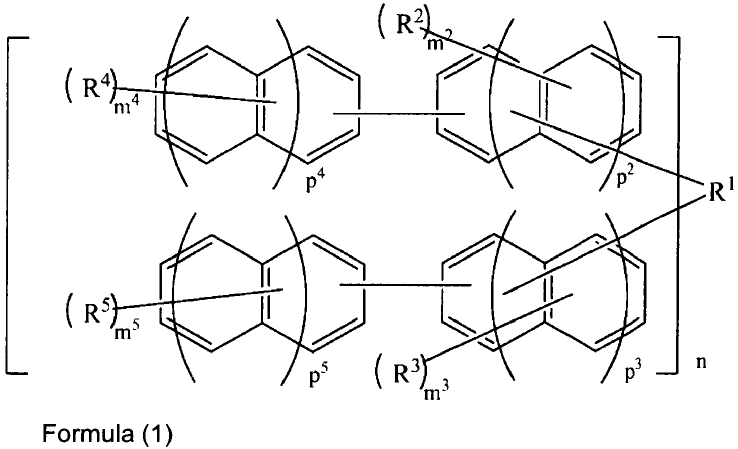 wherein R¹ is a 2n-valent group of 1 to 30 carbon atoms; R² to R⁵ are each independently an alkyl group of 1 to 10 carbon atoms, an aryl group of 6 to 10 carbon atoms, an alkenyl group of 2 to 10 carbon atoms, an alkoxy group of 1 to 30 carbon atoms, a halogen atom, a thiol group, or a hydroxyl group, wherein at least one of R⁴ and/or at least one of R⁵ is one or more kinds selected from a hydroxyl group and a thiol group; m² and m³ are each independently an integer of 0 to 8; m⁴ and m⁵ are each independently an integer of 0 to 9, wherein m⁴ and m⁵ are not 0 at the same time; n is an integer of 1 to 4; and p² to p⁵ are each independently an integer of 0 to 2.

2. The radiation-sensitive composition according to claim 1, wherein in the formula (1), at least one selected from R¹ to R⁵ is a group containing an iodine atom.

3. The radiation-sensitive composition according to claim 1 or 2, wherein in the formula (1), at least one of R² and/or at least one of R³ is one or more kinds selected from a hydroxyl group and a thiol group.

4. The radiation-sensitive composition according to any one of claims 1 to 3, wherein the compound represented by the formula (1) is a compound represented by the following formula (la): 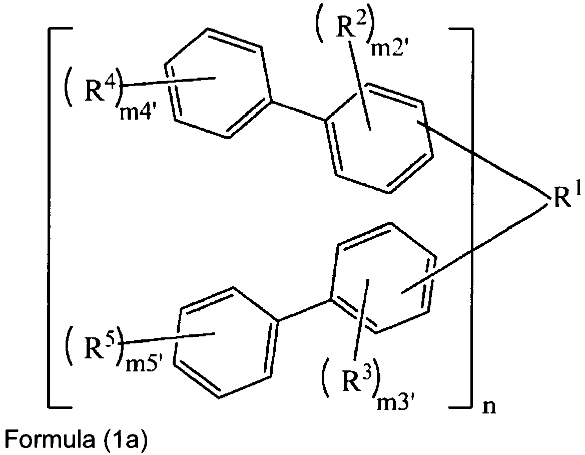 wherein R¹ to R⁵ and n are as defined in the description of the above formula (1) ; m^{2'} and m^{3'} are each independently an integer of 0 to 4; and m^{4'} and m^{5'} are each independently an integer of 0 to 5, wherein m^{4'} and m^{5'} are not 0 at the same time.

5. The radiation-sensitive composition according to claim 4, wherein the compound represented by the formula (1a) is a compound represented by the following formula (1b) : 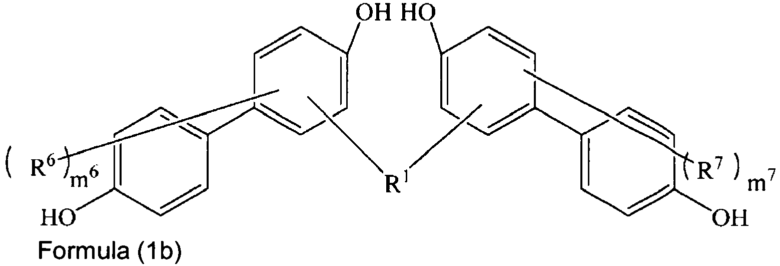 wherein R¹ is as defined in the description of the above formula (1); R⁶ and R⁷ are each independently an alkyl group of 1 to 10 carbon atoms, an aryl group of 6 to 10 carbon atoms, an alkenyl group of 2 to 10 carbon atoms, an alkoxy group of 1 to 30 carbon atoms, a halogen atom, or a thiol group, wherein at least one selected from R¹, R⁶, and R⁷ is a group containing an iodine atom; and m⁶ and m⁷ are each independently an integer of 0 to 7.

6. The radiation-sensitive composition according to claim 5, wherein the compound represented by the formula (lb) is a compound represented by the following formula (1c) : 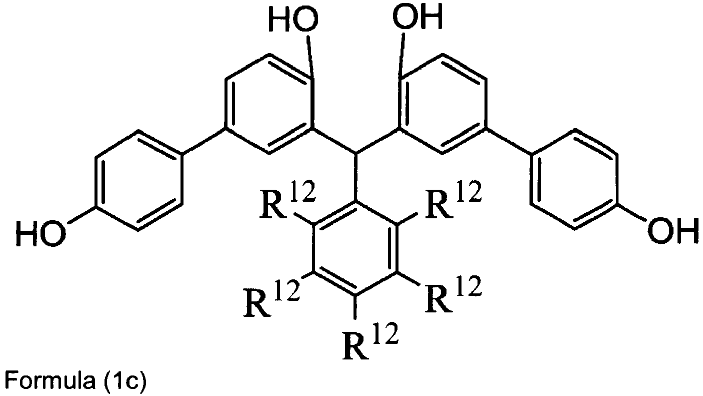 wherein R¹² are each independently a hydrogen atom, a cyano group, a nitro group, a heterocyclic group, a halogen atom, a linear aliphatic hydrocarbon group of 1 to 20 carbon atoms, a branched aliphatic hydrocarbon group of 3 to 20 carbon atoms, a cyclic aliphatic hydrocarbon group of 3 to 20 carbon atoms, an aryl group of 6 to 20 carbon atoms, an alkenyl group of 2 to 10 carbon atoms, an alkoxy group of 1 to 30 carbon atoms, a thiol group, or a hydroxyl group, wherein at least one of R¹² is a group containing an iodine atom.

7. The radiation-sensitive composition according to claim 6, wherein the compound represented by the formula (1c) is a compound represented by the following formula (1d) : 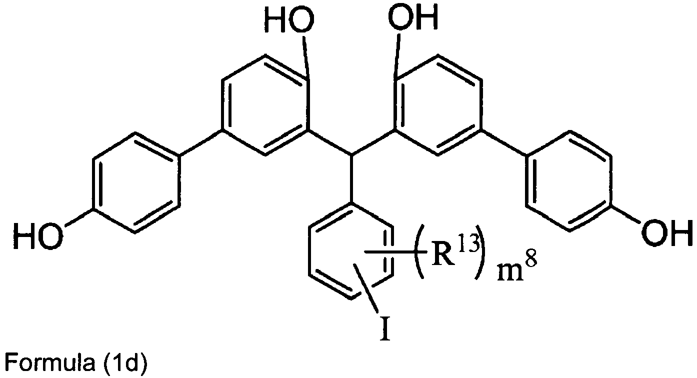 wherein R¹³ are each independently a cyano group, a nitro group, a heterocyclic group, a halogen atom, a linear aliphatic hydrocarbon group of 1 to 20 carbon atoms, a branched aliphatic hydrocarbon group of 3 to 20 carbon atoms, a cyclic aliphatic hydrocarbon group of 3 to 20 carbon atoms, an aryl group of 6 to 20 carbon atoms, an alkenyl group of 2 to 10 carbon atoms, an alkoxy group of 1 to 30 carbon atoms, a thiol group, or a hydroxyl group; and m⁸ is an integer of 0 to 4.

8. The radiation-sensitive composition according to any one of claims 1 to 7, wherein the components except for the solvent (C) comprise resist base material (A)/optically active diazonaphthoquinone compound (B)/optional component (D) at a ratio of 1 to 99/99 to 1/0 to 98 in % by mass based on the components except for the solvent (C).

9. The radiation-sensitive composition according to any one of claims 1 to 8, wherein the radiation-sensitive composition is capable of forming an amorphous film by spin coating.

10. The radiation-sensitive composition according to claim 9, wherein the dissolution rate of the amorphous film in a developing solution at 23°C is 5 angstrom/sec or less.

11. The radiation-sensitive composition according to claim 10, wherein the dissolution rate of the amorphous film irradiated with g-ray, h-ray, i-ray, KrF excimer laser, ArF excimer laser, extreme ultraviolet, electron beam, or X-ray or the amorphous film heated at 20 to 500°C, in a developing solution at 23°C is 10 angstrom/sec or more.

12. An amorphous film obtained using the radiation-sensitive composition according to any one of claims 1 to 11.

13. A method for forming a resist pattern, comprising the steps of: coating a substrate with the radiation-sensitive composition according to any one of claims 1 to 11, thereby forming a resist film; exposing the resist film; and developing the exposed resist film.
